(19) Europäisches Patentamt / European Patent Office / Office européen des brevets

(11) **EP 4 241 796 B1**

(12) **EUROPEAN PATENT SPECIFICATION**

(45) Date of publication and mention
of the grant of the patent:
**17.06.2026 Bulletin 2026/25**

(21) Application number: **21889223.0**

(22) Date of filing: **04.11.2021**

(51) International Patent Classification (IPC):
*A61L 27/04* (2006.01)    *A61L 31/02* (2006.01)
*A61K 6/58* (2020.01)     *A61K 6/70* (2020.01)
*A61C 8/00* (2006.01)     *A61L 27/06* (2006.01)
*A61L 27/38* (2006.01)    *A61L 27/40* (2006.01)
*A61L 27/50* (2006.01)    *A61L 27/30* (2006.01)

(52) Cooperative Patent Classification (CPC):
**A61L 31/022; A61K 6/58; A61L 27/04;
A61L 27/045; A61L 27/06; A61L 27/306;
A61L 27/50; A61L 31/088;** A61C 8/0012;
A61L 2420/00; A61L 2420/06; A61L 2430/02;
A61L 2430/12

(86) International application number:
**PCT/JP2021/040544**

(87) International publication number:
**WO 2022/097670 (12.05.2022 Gazette 2022/19)**

(54) **BIOCOMPATIBLE MATERIAL AND METHOD OF PRODUCING SAME**

BIOKOMPATIBLES MATERIAL UND VERFAHREN ZUR HERSTELLUNG DAVON

MATÉRIAU BIOCOMPATIBLE ET SON PROCÉDÉ DE PRODUCTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.11.2020 JP 2020184954
25.12.2020 JP 2020217810**

(43) Date of publication of application:
**13.09.2023 Bulletin 2023/37**

(73) Proprietor: **Maruemu Works Co., Ltd
Osaka-shi, Osaka 542-0086 (JP)**

(72) Inventors:
• **YAMANAKA, Shigeru
Daitou-shi, Osaka 574-0015 (JP)**
• **NARITA, Kengo
Daitou-shi, Osaka 574-0015 (JP)**

(74) Representative: **Meissner Bolte Partnerschaft
mbB
Patentanwälte Rechtsanwälte
Postfach 86 06 24
81633 München (DE)**

(56) References cited:
**WO-A1-2008/035948**

• **SARAH ZAATREH: "Fast corroding, thin
magnesium coating displays antibacterial
effects and low cytotoxicity", BIOFOULING,, vol.
33, no. 4, 1 January 2017 (2017-01-01), pages 294
- 305, XP009544099, ISSN: 1026-7867, DOI:
10..1080/08927014.2017.1303832**
• **IBASCO, S. ; TAMIMI, F. ; MESZAROS, R. ;
NIHOUANNEN, D.L. ; VENGALLATORE, S. ;
HARVEY, E. ; BARRALET, J.E.: "Magnesium-
sputtered titanium for the formation of bioactive
coatings", ACTA BIOMATERIALIA, ELSEVIER,
AMSTERDAM, NL, vol. 5, no. 6, 1 July 2009
(2009-07-01), AMSTERDAM, NL, pages 2338 -
2347, XP026161800, ISSN: 1742-7061, DOI:
10.1016/j.actbio.2009.03.006**

EP 4 241 796 B1

- AYUKAWA YASUNORI, ATSUTA IKIRU, TSURUTA KATSUHIRO, MATSUSHITA YASUYUKI, KOYANO KIYOSHI: "State-of-the-art of the Surface Modification of Oral Implants", HYOMEN GIJUTSU - JOURNAL OF THE SURFACE FINISHING SOCIETY OFJAPAN, HYOMEN GIJUTSU KYOKAI, TOKYO, JP, vol. 67, no. 6, 1 June 2016 (2016-06-01), JP
, pages 297 - 301, XP055928421, ISSN: 0915-1869, DOI: 10.4139/sfj.67.297
- HORIUCHI, KATSUHIRO: "Relation between the Shape/Surface of the Implant Collar and Peri-Implantitis", JOURNAL OF JAPANESE SOCIETY OF ORAL IMPLANTOLOGY, vol. 29, no. 4, 31 December 2016 (2016-12-31), JP
, pages 208 - 218, XP009536431, ISSN: 0914-6695, DOI: 10.11237/jsoi.29.208
- HARRISON RICHARD; MARADZE DIANA; LYONS SIMON; ZHENG YUFENG; LIU YANG: "Corrosion of magnesium and magnesium–calcium alloy in biologically-simulated environ", PROGRESS IN NATURAL SCIENCE: MATERIALS INTERNATIONAL, ELSEVIER, AMSTERDAM, NL, vol. 24, no. 5, 29 October 2014 (2014-10-29), AMSTERDAM, NL, pages 539 - 546, XP029105305, ISSN: 1002-0071, DOI: 10.1016/j.pnsc.2014.08.010

**Description**

Technical Field

The present invention relates to a biocompatible material comprising a film comprised of magnesium and optional calcium. In particular, the present invention relates to a biocompatible material comprising a film comprised of magnesium and optional calcium, having a predetermined surface roughness, a predetermined adhesion and/or a predetermined hardness.

The present invention also relates to a method of manufacturing the biocompatible material.

Background Art

The treatment of teeth by implants has attracted attention as a type of treatment method for tooth loss for young people to elderly people.

Metallic titanium, a titanium alloy, or ceramic zirconia, that does not relatively adversely affect a living body, is used as a material of a dental root implant.

It is required to shorten a period between the placement of the root implant and the functioning of the root for the patient to be able to bite, that is, a time until secondary fixation in which bone cells are formed around the dental root implant. The above-mentioned implant materials for dental roots such as titanium alone have not been able to meet the demand for shortening the time to secondary fixation, and improvement thereof is required.

For example, Non-Patent Documents 1 and 2 show that the formation of apatite on the surface of a dental root implant is effective for the formation of surrounding bone, and disclose that apatite is applied on the surface of a dental root implant before implantation, to form a film, or that apatite is sputtered to form a film.

Patent Documents 1 and 2 disclose that apatite is similarly formed on a titanium implant. The documents also disclose that a calcium-containing film was chemically formed on the implant adhesion, or that apatite is formed on the calcium-containing film as an intermediate layer, to enhance the adhesiveness.

Non-Patent Document 3 discloses that it is effective to generate an appropriate pressure between a dental root implant and the alveolar bone in order to promote secondary fixation. Therefore, Non-Patent Document 3 discloses that it is necessary to clamp the dental root implant with an appropriate torque at the time of clamping upon initial fixing. However, since the above-described apatite films formed before implantation described in Non-Patent Documents 1 and 2, Patent Documents 1 and 2 have low hardness and/or insufficient adhesiveness, the films are easily peeled off at the time of clamping and embedding so that functions thereof cannot be sufficiently fulfilled.

Patent Document 3 discloses that metal is used for a core body (substrate) of an implant, and that a bioactive glass film including oxides of silicon, sodium, magnesium, calcium, and potassium is formed on a surface thereof instead of apatite, in order to simultaneously realize maintenance of strength of the implant and improvement of a bonding function with a bone. However, since the film is made of inorganic glass or an inorganic compound, the film has hardness but a drawback of being brittle.

Patent Document 4 attempts to promote bone formation by appropriately roughening the surface of a dental root implant made of ceramics such as zirconia. However, in this case, it is possible to perform clamping regardless of the peeling of the film, but the bone formation is not sufficient.

Patent Document 5 discloses that a dental root implant is covered with a protective film in order to take advantage of an active surface of a base material to protect the implant. The protective film is designed to disappear after implantation for the purpose of maintaining aesthetics, but the film has no component or function for promoting bone formation.

As with the case of Patent Document 3, Patent Document 6 discloses that a protective film is formed on the surface for the purpose of protecting the active surface of a base material for a dental root implant, and that a component of the film is formed of a salt that generates cations of sodium, potassium, magnesium, and calcium. However, in salts, the strength and adhesiveness of the film required at the time of implantation are not sufficient.

Non-Patent Document 4 discloses an orthopedic implant in which a metallic pure magnesium film is formed on the surface by an ion plating method instead of apatite. Although the effect of magnesium ions on bone formation has been shown, the ability to form apatite is not sufficient because calcium, which is a main component of apatite, does not exist. Further, in the arc ion plating used in the Non-Patent Document **4,** since the particles which is to be the film are large, the surface roughness is large, and thus, it is difficult to control the roughness. More, even if calcium is further used as a target to form an alloy in this method, an intermetallic compound is formed in the film or the like, and thus the film becomes brittle, since the film is made of the cluster of the alloy.

Non-Patent Document 5 proposes that a ternary alloy of Mg-Ca-Zn is useful as a core body (substrate) of an implant as a biodegrading material. When Mg is considered as a main component, if Ca is added to form an alloy, the solid solution limit is 1% or less, and, if Ca is added more than 1%, an intermetallic compound such as $Mg_2Ca$ is formed. Therefore, the content of Ca in such an alloy is at most 5%, and there is a possibility that an intermetallic compound may remain in the body in addition to Mg ions and Ca ions.

Non-Patent Document 6 discloses that Mg-Ca-Zn to which Ca is added up to 15% is manufactured as an amorphous ribbon at a high temperature by high-speed rotary quenching method (spinning method). However, since about 7% of the intermetallic compound is formed, it is not easy to produce uniform amorphous, and thus, the shape and the size are limited. Further, Non-Patent Document 7 discloses that a Ca-Mg-Zn alloy including Ca as main metal is attempted to be manufactured by a similar method in order to enhance the effect of Ca. However, the method cannot achieve uniform amorphous formation.

Non-Patent Documents 8 and 9 disclose that a Mg-Ca-Zn ternary system in which Ca is added from 4 % by weight to 24 % by weight is manufactured as a thin film in an amorphous state by sputtering. Non-Patent Document 9 discloses that these thin films contain at least 30 % by weight of Zn in order to easily form an amorphous state. However, the document also disclose that Zn ions exhibit cytotoxicity, and thus that the higher amount of Zn exhibits the stronger cytotoxicity.

Patent document 7 discloses a hip prosthesis comprising a titanium substrate coated with an alloy of Mg-Ca comprising 0.8, 5, 10.5, 23 or 33% Ca and the balance being pure Mg (purity 99.995%). The thickness of the coating is 5 micrometers.

In order to stably manufacture an amorphous metal, a multicomponent alloy (having components of three or more elements) capable of lowering the liquid phase temperature is generally required. However, conventional method cannot manufacture amorphous metal consisting essentially of two components, Mg-Ca.

Prior Art Document

Non-Patent Document

Non-Patent Document 1: KYOCERA Corporation POIEX/HACEX Catalogue.
Non-Patent Document 2: Kyousuke UEDA, Materia Japan, Volume 51, No. 9 (2012).
Non-Patent Document 3: Implant Journal 2017, Autumn, p.8
Non-Patent Document 4: X. Li et al., Scientific Reports, 7: 40755 (2017).
Non-Patent Document 5: J. Hofsteter et al., JOM, Vol. 68, No. 4 (2014), p. 566-572.
Non-Patent Document 6: S. Paul al., S25 Materialia (2020).
Non-Patent Document 7: K. Saksl et al., J. Alloys and Compounds 801 (2019) p.651-657.
Non-Patent Document 8: J. Liu et al., J. Alloys and Compounds 742 (2018) p. 524-535.
Non-Patent Document 9: J. Li et al., Chemical Communications 53 (2017) p.8288-8291

Patent Document

Patent Document 1: WO2009/147819.
Patent Document 2: JP Patent No. 4425198.
Patent Document 3: JP-B Hei3-2540.
Patent Document 4: WO2016/189099A1.
Patent Document 5: WO2020/099334A2.
Patent Document 6: EP 1847278 A1.
Patent Document 7: WO 2008/035948 A1.

Summary of Invention

Problems to be solved by the Invention

An object of the present invention is to provide a biocompatible material comprising a film that realizes bone formation around the biocompatible material in a relatively short period of time.

Further, other than or in addition to the above object, an object of the present invention is to provide a biocompatible material comprising a film with a relatively smooth surface roughness, suitable for implantation or clamping at an appropriate implanting torque.

More, other than or in addition to the above objects, an object of the present invention is to provide a biocompatible material comprising a film with adhesiveness and/or hardness that can withstand implantation or clamping at an appropriate implanting torque.

Further, other than or in addition to the above objects, an object of the present invention is to provide a method of manufacturing the biocompatible material.

Means for Solving Problems

The present inventors have found the following inventions:
a biocompatible material as defined in claim 1; a method of manufacturing a biocompatible material according to claim 9.
<A4> A biocompatible material having a film comprised of magnesium and optional calcium,
wherein the film comprises 0 to 40 % by weight of calcium, where a total weight of magnesium and calcium is 100 % by weight, and the film has any one or two, or all of following characteristics i) to iii):

i) hardness obtained by an indentation test is 0.4 GPa or more, preferably 0.9 GPa or more, more preferably 1.2 GPa or more;

ii) a relationship Wc/t between a critical load Wc (N) and a film thickness t ($\mu$m) is 1 N/$\mu$m or more, preferably 2 N/$\mu$m or more; and

iii) a critical shear stress at an interface between the film and a substrate is 80 MPa or more, preferably 160 MPa or more.

<A5> In the above item <A4>,

a) the arithmetic mean surface height Sa1 of the surface roughness of the film may be 2 $\mu$m or less, preferably 1 $\mu$m or less, and/or

b) a difference between an arithmetic mean surface height Sa1 of surface roughness of the film and an arithmetic mean surface height Sa2 of surface roughness of a surface which does not comprise the film may be 300 nm or less, preferably 200 nm or less, more preferably 150 nm or less.

<A6> In any one of the above items <A1> to <A5>, an average thickness of the film may be 0.10 to 30 $\mu$m. In particular, the average thickness of the film corresponding to a contact portion with the bone and the vicinity thereof may be 0.10 to 30 $\mu$m, preferably 0.20 to 20 $\mu$m, and more preferably 0.40 to 15 $\mu$m.

<A7> In any one of the above items <A1> to <A6>, the film may consist essentially of magnesium.

<A8> In any one of the above items <A1> to <A6>, the film may consist of magnesium.

<A9> In any one of the above items <A1> to <A6>, the film may be comprised of magnesium and calcium, the film may comprise more than 0 % by weight and not more than 40 % by weight, preferably 0.8 to 35 % by weight more preferably 5 to 30 % by weight, and most preferably 15 to 25 % by weight of calcium.

<A10> In the above item <A9>, the film may consist essentially of magnesium and calcium.

<A11> In the above item <A9>, the film may consist of magnesium and calcium.

<A12> In any one of the above items <A9> to <A11>, the film may be free from $Mg_2Ca$.

<A13> In any one of the above items <A9> to <A12>, the film may comprise an amorphous portion.

<A14> In any one of the above items <A9> to <A12>, the film may consist essentially of amorphous, preferably may consist of amorphous.

<A15> In any one of the above items <A1> to <A14>, the biocompatible material may comprise a biocompatible substrate, and the biocompatible substrate may be at least one selected from the group consisting of pure titanium, zirconia, a cobalt-chromium alloy, stainless steel, and a titanium alloy.

<A16> In any one of the above items <A1> to <A15>, the biocompatible material may be one selected from the group consisting of an artificial bone material, an intraosseous fixture material, a dental implant material, an orthodontic anchor screw material, a medullary nail material, and an interbody fixation material. For example, the biocompatible material may be an artificial bone, a pin, wire, a bolt, a screw, a washer, a medullary nail, and a vertebral body spacer, and the like.

<A17> In any one of the above items <A1> to <A16>, a shape of the biocompatible material may be one selected from the group consisting of a cylindrical shape, a truncated cone shape, a conical shape, a shape having a screw-shaped threaded portion in a part of the shape, a rectangular parallelepiped and a cube, a block shape such as a rectangular parallelepiped and a cube having a partially inclined surface, and a wedge shape.

<A18> A method of manufacturing a biocompatible material comprising a film comprised of magnesium and optional calcium, comprising the steps of:

(A) preparing a biocompatible substrate;
(B) preparing a sputtering target comprised of magnesium and optional calcium;

(C) cleaning a surface of the biocompatible substrate in vacuum; and

(D) sputtering by using the sputtering target and setting a temperature of the biocompatible substrate obtained in Step (C) at 130°C or lower, preferably 90°C or lower, more preferably 60°C or lower, to form the film comprised of magnesium and optional calcium on the biocompatible substrate, wherein the film comprises 0 to 40 % by weight of calcium where a total weight of magnesium and calcium is 100 % by weight.

<A19> In the above item <A18>,

a) the arithmetic mean surface height Sa1 of the surface roughness of the film may be 2 $\mu$m or less, preferably 1 $\mu$m or less, and/or

b) a difference between an arithmetic mean surface height Sa1 of surface roughness of the film and an arithmetic mean surface height Sa2 of surface roughness of a surface which does not comprise the film may be 300 nm or less, preferably 200 nm or less, more preferably 150 nm or less.

<A20> In the above item <A18> or <A19>, the film may have any one or two, or all of following characteristics i) to iii):

i) hardness obtained by an indentation test is 0.4 GPa or more, preferably 0.9 GPa or more, more preferably 1.2 GPa or more;

ii) a relationship Wc/t between a critical load Wc (N) and a film thickness t ($\mu$m) is 1 N/$\mu$m or more, preferably 2 N/$\mu$m or more; and

iii) a critical shear stress at an interface between the film and a substrate is 80 MPa or more, preferably 160 MPa or more.

<A21> In any one of the above items <A18> to <A20>, an average thickness of the film may be 0.10 to 30 $\mu$m, preferably 0.20 to 20 $\mu$m, and more preferably 0.40 to 15 $\mu$m.

<B1> A biocompatible material comprising a film comprised of magnesium and optional calcium, wherein the film comprises 0 to 40 % by weight of calcium, where a total weight of magnesium and calcium is 100 % by weight, and an arithmetic mean surface height Ra1 of line roughness of the film is 2 $\mu$m or less, preferably 1 $\mu$m or less.

<B2> A biocompatible material comprising a film comprised of magnesium and optional calcium, wherein the film comprises 0 to 40 % by weight of calcium, where a total weight of magnesium and calcium is 100 % by weight, and a difference between an arithmetic mean surface height Ra1 of line roughness of the film and an arithmetic mean surface height Ra2 of line roughness of a surface which does not comprise the film is 300 nm or less, preferably 200 nm or less, preferably 150 nm or less.

<B3> In the above item <B2>, an arithmetic mean surface height Ra1 of line roughness of the film is 2 $\mu$m or less, preferably 1 $\mu$m or less.

<B4> In any one of the above items <B1> to <B3>, the film may have any one or two, or all of following characteristics i) to iii):

i) hardness obtained by an indentation test is 0.4 GPa or more, preferably 0.9 GPa or more, more preferably 1.2 GPa or more;

ii) a relationship Wc/t between a critical load Wc (N) and a film thickness t ($\mu$m) is 1 N/$\mu$m or more, preferably 2 N/$\mu$m or more; and

iii) a critical shear stress at an interface between the film and a substrate is 80 MPa or more, preferably 160 MPa or more.

<B5> In any one of the above items <B1> to <B4>,

c) an arithmetic mean surface height Sa1 of surface roughness of the film may be 2 $\mu$m or less, preferably 1 $\mu$m or less; and/or

d) a difference between the arithmetic mean surface height Sa1 of the surface roughness of the film and an arithmetic mean surface height Sa2 of surface roughness of a surface which does not comprise the film may be 300 nm or less, preferably 200 nm or less, more preferably 150 nm or less.

<B6> In any one of the above items <B1> to <B5>, an average thickness of the film may be 0.10 to 30 $\mu$m. In particular, the average thickness of the film corresponding to a contact portion with the bone and the vicinity thereof may be 0.10 to 30 $\mu$m, preferably 0.20 to 20 $\mu$m, and more preferably 0.40 to 15 $\mu$m.

<B7> In any one of the above items <B1> to <B6>, the film may consist essentially of magnesium.

<B8> In any one of the above items <B1> to <B6>, the film may consist of magnesium.

<B9> In any one of the above items <B1> to <B6>, the film may be comprised of magnesium and calcium, the film may comprise more than 0 % by weight and not more than 40 % by weight, preferably 0.8 to 35 % by weight more preferably 5 to 30 % by weight, and most preferably 15 to 25 % by weight of calcium.

<B10> In the above item <B9>, the film may consist essentially of magnesium and calcium.

<B11> In the above item <B9>, the film may consist of magnesium and calcium.

<B12> In any one of the above items <B9> to <B11>, the film may be free from $Mg_2Ca$.

<B13> In any one of the above items <B9> to <B12>, the film may comprise an amorphous portion.

<B14> In any one of the above items <B9> to <B12>, the film may consist essentially of amorphous, preferably may consist of amorphous.

<B15> In any one of the above items <B1> to <B14>, the biocompatible material may comprise a biocompatible substrate, and the biocompatible substrate may be at least one selected from the group consisting of pure titanium, zirconia, a cobalt-chromium alloy, stainless steel, and a titanium alloy.

<B16> In any one of the above items <B1> to <B15>, the biocompatible material may be one selected from the group consisting of an artificial bone material, an intraosseous fixture material, a dental implant material, an orthodontic anchor screw material, a medullary nail material, and an interbody fixation material. For example, the biocompatible material may be an artificial bone, a pin, wire, a bolt, a screw, a washer, a medullary nail, and a vertebral body spacer, and the like.

<B17> In any one of the above items <B1> to <B16>, a shape of the biocompatible material may be one selected from the group consisting of a cylindrical shape, a truncated cone shape, a conical shape, a shape having a screw-shaped threaded portion in a part of the shape, a rectangular parallelepiped and a cube, a block shape such as a rectangular parallelepiped and a cube having a partially inclined surface, and a wedge shape.

<B18> A method of manufacturing a biocompatible material comprising a film comprised of magnesium and optional calcium, comprising the steps of:

(A) preparing a biocompatible substrate;
(B) preparing a sputtering target comprised of magnesium and optional calcium;
(C) cleaning a surface of the biocompatible substrate in vacuum; and
(D) sputtering by using the sputtering target and setting a temperature of the biocompatible substrate obtained in Step (C) at 130°C or lower, preferably 90°C or lower, more preferably 60°C or lower, to form the film comprised of magnesium and optional calcium on the biocompatible substrate, wherein the film comprises 0 to 40 % by weight of calcium where a total weight of magnesium and calcium is 100 % by weight,

a) an arithmetic mean surface height Ra1 of line roughness of the film is 2 μm or less, preferably 1 μm or less; and
b) a difference between the arithmetic mean surface height Ra1 of the line roughness of the film and an arithmetic mean surface height Ra2 of line roughness of a surface which does not comprise the film is 300 nm or less, preferably 200 nm or less, more preferably 150 nm or less.

<B19> In the above item <B18>,

c) the arithmetic mean surface height Sa1 of the surface roughness of the film may be 2 μm or less, preferably 1 μm or less, and/or
d) a difference between an arithmetic mean surface height Sa1 of surface roughness of the film and an arithmetic mean surface height Sa2 of surface roughness of a surface which does not comprise the film may be 300 nm or less, preferably 200 nm or less, more preferably 150 nm or less.

<B20> In the above item <B18> or <B19>, the film may have any one or two, or all of following characteristics i) to iii):

i) hardness obtained by an indentation test is 0.4 GPa or more, preferably 0.9 GPa or more, more preferably 1.2 GPa or more;
ii) a relationship Wc/t between a critical load Wc (N) and a film thickness t (μm) is 1 N/μm or more, preferably 2 N/μm or more; and
iii) a critical shear stress at an interface between the film and a substrate is 80 MPa or more, preferably 160 MPa or more.

Effects of the Invention

The present invention can provide a biocompatible material comprising a film that realizes bone formation around the biocompatible material in a relatively short period of time.

Further, other than or in addition to the above effect, the present invention can provide a biocompatible material comprising a film with a relatively smooth surface roughness, suitable for implantation or clamping at an appropriate implanting torque.

More, other than or in addition to the above effects, the present invention can provide a biocompatible material comprising a film with adhesiveness and/or hardness that can withstand implantation or clamping at an appropriate implanting torque.

Further, other than or in addition to the above effects, the present invention can provide a method of manufacturing the biocompatible material.

Brief Description of Drawings

Fig. 1 is an illustrative diagram illustrating how to measure the roughness of the threaded portion of a sample in a case of the sample having a cylindrical shape or a truncated cone shape and having a threaded portion of a screw.

Fig. 2 is an illustrative diagram illustrating how to measure the roughness of the threaded portion of a sample in a case of the sample having a cylindrical shape or a truncated cone shape and having a threaded portion of a screw.

Fig. 3 is a graph illustrating measurement results of arithmetic mean surface roughness for the films A1-C0, A1-C10, A1-C20, and A1-C30 formed by using titanium as plate-shaped substrates (hatched bars show results for Ra1, and unhatched bars show results for Ra2).

Fig. 4 is a graph illustrating measurement results of arithmetic mean surface roughness for the films A2-C0, A2-C10, A2-C20, and A2-C30 formed by using zirconia as plate-shaped substrates (hatched bars show results for Ra1, and unhatched bars show results for Ra2).

Fig. 5 is a graph illustrating measurement results of arithmetic mean surface roughness for the films A1-C0, A1-C10, A1-C20, and A1-C30 formed by using titanium as plate-shaped substrates (hatched bars show results for Sa1, and unhatched bars show results for Sa2).

Fig. 6 is a graph illustrating measurement results of arithmetic mean surface roughness for the films A2-C0, A2-C10, A2-C20, and A2-C30 formed by using zirconia as plate-shaped substrates (hatched bars show results for Sa1, and unhatched bars show results for Sa2).

Fig. 7 illustrates scanning electron microscope images (SEM images) of films in a cross-sectional direction for the films AC1-C0, AC1-C10, AC1-C20, and AC1-C30 formed by using a glass substrate.

Fig. 8 illustrates scanning electron microscope images of surfaces of films A1-C0, A1-C10, A1-C20, and A1-C30 formed by using titanium as plate-shaped substrates.

Fig. 9 illustrates scanning electron microscope images of surfaces of films A2-C0, A2-C10, A2-C20, and A2-C30 formed by using zirconia as plate-shaped substrates.

Fig. 10 is a graph illustrating results of X-ray diffraction analysis for films AC1-C0, AC1-C10, AC1-C20, and AC1-C30 formed by using a glass substrate.

Fig. 11 is a graph illustrating results of X-ray diffraction analysis for films A1-C0, A1-C10, A1-C20, and A1-C30 formed by using titanium as plate-shaped substrates.

Fig. 12 is a graph illustrating results of X-ray diffraction analysis for films A2-C0, A2-C10, A2-C20, and A2-C30 formed by using zirconia as plate-shaped substrates.

Fig. 13 is a graph illustrating results of a hardness test by a nanoindentation method using an apparatus (an ultra-micro indentation hardness tester "ENT-1100A" of ELIONIX INC.) under conditions: indenter: Berkovich indenter; an indentation load: 20 mN; and retention time: 0 second.

Fig. 14 is views illustrating SEM images of appearances near critical loads after scratch tests in a direction parallel to grinding marks for films A1-C0, A1-C10, A1-C20, and A1-C30 formed by using titanium as plate-shaped substrates.

Fig. 15 is views illustrating SEM images of appearances near critical loads after scratch tests in a direction parallel to grinding marks for films A2-C0, A2-C10, A2-C20, and A2-C30 formed by using zirconia as plate-shaped substrates.

Fig. 16 is a graph illustrating measurement results for finding loads when an indenter almost reaches substrates, that is, critical loads of films in scratch tests in a direction parallel or perpendicular to grinding marks of films A1-C0, A1-C10, A1-C20, and A1-C30 formed by using titanium as plate-shaped substrates.

Fig. 17 illustrates measurement results for finding loads when an indenter almost reaches substrates, that is, critical loads of films in scratch tests in a direction parallel or perpendicular to grinding marks of films A2-C0, A2-C10, A2-C20, and A2-C30 formed by using zirconia as plate-shaped substrates.

Fig. 18 is a graph illustrating yield shear stresses of A1-C0 to A1-C30 and A2-C0 to A2-C30 converted from the hardness of the films of A1-C0 to A1-C30 and A2-C0 to A2-C30.

Fig. 19 is a schematic view illustrating a method of obtaining a critical load in a scratch test when a substrate has a cylindrical shape.

Fig. 20 is views illustrating SEM images of films newly formed on substrates by immersing films A1-C0, A1-C10, A1-C20, and A1-C30 formed by using titanium as plate-shaped substrates in HBSS for one week.

Fig. 21 is views illustrating SEM images of films newly formed on substrates by immersing films A2-C0, A2-C10, A2-C20, and A2-C30 formed by using zirconia as plate-shaped substrates in HBSS for one week.

Fig. 22 is a graph illustrating results of X-ray diffraction (XRD) analysis by a thin film method for a plate-shaped substrate A1 using titanium and films A1-C0, A1-C10, A1-C20, and A1-C30 formed by using the substrate.

Fig. 23 is a graph illustrating results of X-ray diffraction (XRD) analysis by a thin film method for a plate-shaped substrate A2 using zirconia and films A2-C0, A2-C10, A2-C20, and A2-C30 formed by using the substrate.

Fig. 24 is a graph illustrating comparison of an arithmetic mean height Ra1 of line roughness between a sample AIPC1-C0 formed on a glass substrate by using pure magnesium by arc ion plating and films AC1-C0, AC1-C10, AC1-C20, and AC1-C30 formed on a glass substrate by using pure magnesium by sputtering.

Fig. 25 is a view illustrating a comparison of results of an XRD and a scratch test between the film A2-C20 formed by using zirconia as a plate-shaped substrate and the film A2-C20-H obtained by subjecting the film to a heat treatment.

Description of Embodiments

The invention described in the present application (hereinafter, may be abbreviated as the "present invention") is explained hereinafter.

The present application provides a biocompatible material as defined in claim 1. Preferably, the amount of calcium is 0.8 to 35 % by weight, more preferably 5 to 30 % by weight, and most preferably 15 to 25 % by weight, when calcium is comprised.

Further, the amount of calcium may be more preferably 5 to 35 % by weight, more preferably 15 to 35 % by weight, most preferably 25 to 35 % by weight.

In one aspect, the present invention provides a biocompatible material in which a) an arithmetic mean height Ra1 of line roughness of the film is 2 $\mu$m or less, preferably 1 $\mu$m or less.

Further, in another aspect, the present invention provides a biocompatible material in which b) a difference between the arithmetic mean height Ra1 of the line roughness of the film and an arithmetic mean height Ra2 of line roughness of a surface which does have the film is 300 nm or less, preferably 200 nm or less, more preferably 150 nm or less.

More, in one aspect, the present invention provides a biocompatible material in which c) an arithmetic mean height Sa1 of surface roughness of the film is 2 $\mu$m or less, preferably 1 $\mu$m or less.

Further, in another aspect, the present invention provides a biocompatible material in which d) a difference between the arithmetic mean height Sa1 of the surface roughness of the film and an arithmetic mean height Sa2 of surface roughness of a surface which does not have the film is 300 nm or less, preferably 200 nm or less, more preferably 150 nm or less.

The biocompatible material may have the characteristics of the above a) and/or b) and the characteristics of the above c) and/or d).

More, in the present invention, on still another aspect, the film may have the characteristics of any one or two, or all of the following i) to iii):

i) the hardness obtained by an indentation test is 0.4 GPa or more, preferably 0.9 GPa or more, more preferably 1.2 GPa or more;

ii) a relationship Wc/t between a critical load Wc (N) and a film thickness t ($\mu$m) is 1 N/$\mu$m or more, preferably 2 N/$\mu$m or more; and

iii) a critical shear stress at an interface between the film and the substrate is 80 MPa or more, preferably 160 MPa or more.

The "biocompatibility" in the "biocompatible material" of the present invention refers to a characteristic of being maintained in a living body and not causing a biological safety problem.

<Film>

The biocompatible material of the present invention may have the above-mentioned characteristics a) and/or b), specifically, the arithmetic mean height Ra1 of predetermined line roughness, a predetermined difference between the arithmetic mean heights Ra1 and Ra2 of the line roughness, a predetermined critical load that is an index of predetermined adhesiveness, predetermined hardness, and/or a predetermined critical shear stress at the interface between the film and the substrate.

Further, the biocompatible material of the present invention may have the above-mentioned characteristics c) and/or d), specifically, the arithmetic mean height Sa1 of predetermined surface roughness, a predetermined difference between the arithmetic mean heights Sa1 and Sa2 of the surface roughness, a predetermined critical load that is an index of predetermined adhesiveness, predetermined hardness, and/or a predetermined critical shear stress at the interface between the film and the substrate.

More, the biocompatible material of the present invention may have any one, any two, any three, or all of the characteristics a) to d) described above.

<<Arithmetic Mean Surface Height Ra1 of Line Roughness of Surface having Film>>

The arithmetic mean height Ra1 of the line roughness of the film of the biocompatible material of the present invention may be 2 $\mu$m or less, preferably 1 $\mu$m or less.

The surface of the film, i.e., the arithmetic mean height Ra1 of the line roughness of the film, can be measured in accordance with JIS B 0601: 2013. In the standard, a reference length and an evaluation length are determined according to the classification of the Ra value. For example, in a case of $0.1 < Ra (\mu m) \leq 2$, the reference length and the evaluation length are 0.8 mm and 2.0 mm, respectively. If the reference length cannot be continuously measured due to an interference between a stylus and a measurement object, a reference length of a section lower than an Ra value of the measurement object is used, the number of times of the evaluation length or more in total is measured, and the average value thereof can be set as Ra.

Furthermore, the surface of the biocompatible substrate in the biocompatible material of the present application may be ground in a certain direction or polished all over the surfaces. In the case of polishing all over the surfaces, the roughness of the surface becomes substantially uniform. However, in the case of grinding the surface in a certain direction, when the probe light is deflected along the directional roughness, a measurement error is likely to occur, and the arithmetic mean surface of the line roughness along the direction and the arithmetic mean height of the line roughness in the direction perpendicular to the direction may have different values. When the biocompatible substrate has a cylindrical shape or a threaded shape, the arithmetic mean height of the line roughness of the film in the present application refers to a value measured by moving a probe in a circumferential direction. In the case of other shapes, the biocompatible substrate may be measured in all directions, but for example, the arithmetic mean height refers to a value obtained by measuring the biocompatible substrate in four directions at intervals of 45° and averaging measured values.

<<Arithmetic Mean Height Ra2 of Line Roughness of Surface which does not have the Film>>

In the present application, the "surface which does not have a film" of the "arithmetic mean height Ra2 of the line roughness of the surface which does not have a film" refers to the surface of the substrate before film formation or the surface of the substrate when the film is removed after the film formation.

The film of the present application consists of magnesium and calcium as described above. The film can generally be substantially completely removed by immersion in a weakly acidic aqueous solution or water. Furthermore, the biocompatible substrate in the biocompatible material of the present application is not eroded by the weakly acidic aqueous solution used for removing the film.

Therefore, the arithmetic mean height Ra2 of the line roughness of the surface of the substrate before film formation is substantially the same as an arithmetic mean height Ra2' of the surface of the substrate when the film is removed after the film formation.

Ra2' can also be measured in a manner similar to that of Ra1, in accordance with JIS B 0601: 2013.

In one aspect of the present application, a difference between the arithmetic mean height Ra1 of the line roughness of the film and an arithmetic mean height Ra2 of line roughness of a surface which does not have the film may be 300 nm or less, preferably 200 nm or less, more preferably 150 nm or less.

A method of measuring the arithmetic mean height of the line roughness of a threaded portion of a material which has a generally cylindrical shape or truncated cone shape and having a threaded portion of a screw, such as a dental implant material will be described.

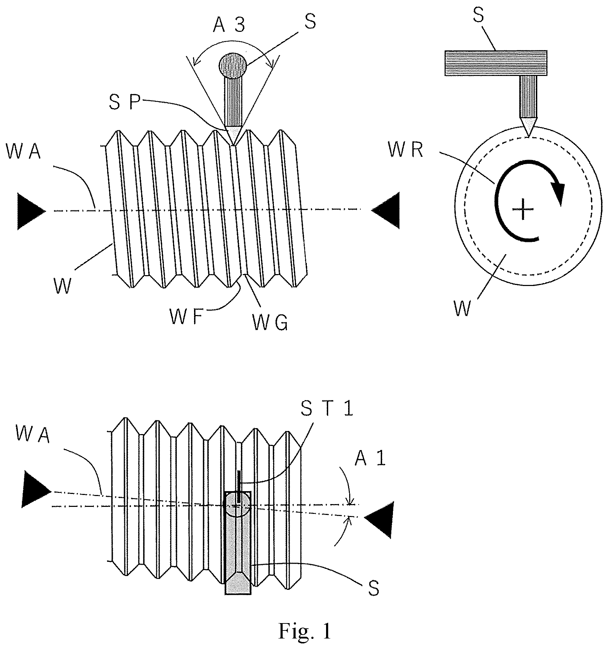

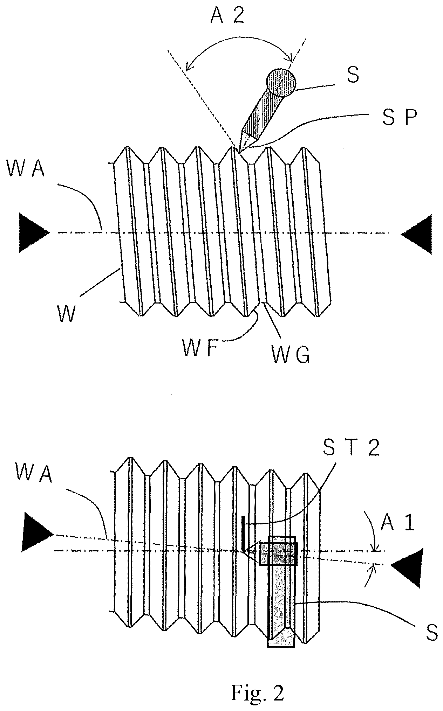

Figs. 1 and 2 illustrate a case where roughness of the threaded portion is measured when a measurement object has a generally cylindrical shape and has a threaded portion of a screw.

The screw is attached to a device having a rotation mechanism by using a chuck or the like such that a thread shaft WA of a threaded portion W serves as a rotation shaft. The measurement can be performed in a manner similar to the measurement on the flat surface: a tip SP of a probe of a stylus S of a roughness measurement device is setted perpendicularly to a valley portion WG of the thread, and a measurement object W is rotated at a constant speed in a rotation direction WR, with keeping the stylus fixed. Furthermore, since there is a lead angle in the thread, it is preferable to perform measurement by skewing the shaft WA by an angle A1 corresponding to the lead angle, in order to prevent the tip SP of the probe from interfering with a thread flank portion WF. As a result, a measurement length ST1 can be measured along the valley WG of the thread.

It is friction on the flank surface of the thread that most influences on an implanting torque upon implanting the screw. Therefore, the roughness of the thread flank surface WF is important. In order to measure this surface, as illustrated in Fig. 2, it is preferable to measure the surface by disposing the stylus S or the rotation shaft WA of the screw in an inclined manner such that an angle A2 between the probe SP and the flank surface becomes a right angle as much as possible. In this way, the measurement length ST2 is measured along the circumferential direction of the flank surface of the thread. In this manner, even in the case of having a screw shape, the line roughness of the valley WG of the thread and the flank surface WF of the thread can be measured according to a desired measurement length (ST1 or ST2) by setting the rotation speed, the rotation radius of the measurement point, and the rotation time.

When the measurement length ST1 or ST2 is large, it is preferable to provide a mechanism for sliding the shaft WA in the traveling direction of the thread (the left direction in Figs. 1 and 2) using a lead screw having the same pitch as the screw to be measured. Further, an angle A3 of the tip SP of the probe is preferably 60 degrees or less in consideration of the width of the thread valley WG so as not to interfere with the ridge or the flank portion WF of the thread.

As described above, even if the measurement object has a substantially cylindrical shape or a truncated cone shape and has the threaded portion of the screw, the line roughness of the threaded portion can be measured.

<<Arithmetic Mean Surface Height Sa1 of Surface Roughness of Surface having Film>>

The arithmetic mean height Sa1 of the surface roughness of the film of the biocompatible material of the present invention may be 2 $\mu$m or less, preferably 1 $\mu$m or less.

The surface having film, i.e., the arithmetic mean height Sa1 of the surface roughness of the film, can be measured in accordance with ISO 25178.

When the surface roughness is measured by laser light, 10,000 or more measurement points can be acquired in a measurement range of 100 $\mu$m $\times$ 100 $\mu$m. The arithmetic mean height can be calculated without using a filter for subjecting a calibration surface leveled by a least squares method to shape removal by a quadratic polynomial and separating wavelength components of a contour curve. Further, an average value may be obtained by totaling measurement distances in one direction and performing the measurement by equal to or more than the number of times to be the evaluation length in accordance with JIS 0633/ISO 4288.

Furthermore, the surface of the biocompatible substrate in the biocompatible material of the present application may be ground in a certain direction or polished all over the surface. In the case of polishing all over the surface, the roughness of the surface becomes substantially uniform. However, in the case of grinding the surface in a certain direction, when the probe light is deflected along the directional roughness, a measurement error is likely to occur, and the arithmetic mean surface along the direction and the arithmetic mean height of the surface roughness in the direction perpendicular to the direction may have different values. In the present application, the arithmetic mean height of the surface roughness of the film refers to a value when the arithmetic mean surface roughness of the surface roughness is measured by deflecting probe light in three directions of parallel, perpendicular, and oblique about 45° with respect to the center line of the shape of the biocompatible material, and the direction in which the roughness is the lowest is measured.

<<Arithmetic Mean Height Sa2 of Surface Roughness of Surface not having Film>>

In the present application, the "surface which does not have a film" of the "arithmetic mean height Sa2 of the surface roughness of the surface which does not have a film" refers to the surface of the substrate before the film formation or the surface of the substrate when the film is removed after the film formation.

The film can generally be substantially completely removed by immersion in a weakly acidic aqueous solution or water. Furthermore, the biocompatible substrate in the biocompatible material of the present application is not eroded by the weakly acidic aqueous solution used for removing the film.

Therefore, the arithmetic mean height Sa2 of the surface roughness of the surface of the substrate before the film formation is substantially the same as an arithmetic mean height Sa2' of the surface of the substrate when the film is removed after the film formation.

Sa2' can also be measured in a manner similar to Sa1, in accordance with ISO 25178.

In one aspect of the present application, a difference between the arithmetic mean height Sa1 of the surface roughness of the film and an arithmetic mean height Sa2 of surface roughness of a surface which does not have the film may be 300 nm or less, preferably 200 nm or less, more preferably 150 nm or less.

In one aspect of the present application, the film may have the characteristics of any one or two, or all of the following i) to iii):

i) the hardness obtained by an indentation test is 0.4 GPa or more, preferably 0.9 GPa or more, more preferably 1.2 GPa or more;
ii) a relationship Wc/t between a critical load Wc (N) and a film thickness t ($\mu$m) is 1 N/$\mu$m or more, preferably 2 N/$\mu$m or more; and
iii) a critical shear stress at an interface between the film and the substrate is 80 MPa or more, preferably 160 MPa or more.

<<Hardness Obtained by Indentation Test>>

In one aspect of the present application, the hardness obtained by an indentation test of the film may be 0.4 GPa or more, preferably 0.9 GPa or more, more preferably 1.2 GPa or more.

In the hardness test of the nanoindentation method, the indentation load may be set so that the indentation depth is 1/5 or less, preferably 1/10 or less of the film thickness. The average value may be obtained by performing measurement 20 times or more, preferably 30 times or more per one sample. For example, the measurement can be performed under the following conditions: an indenter: Berkovich indenter; an indentation load: 20 mN; and retention time: 0 second.

<<Critical Load Obtained by Scratch Test>>

In the present application, the critical load Wc refers to a load when a brittle crack occurs without causing the film to exhibit ductility, a load when the film is partially peeled off, or a load when the film is scraped and the substrate is exposed.

In one aspect of the present application, Wc/t wherein Wc is the critical load (N) and t is the film thickness ($\mu$m)) of the film may be 1 N/$\mu$m or more, preferably 2 N/$\mu$m or more. For example, when the film thickness is 5 $\mu$m, the film may have a critical load of 5 N or more, preferably 10 N or more.

The scratch test can be performed using a device usually used for the scratch test, by using a Rockwell indenter having a diameter of 0.8 mm, for example, in a range in which the load is 0 to 8 kg by weight, to a range in which twice the load at which the substrate is exposed is maximum, a range in which twice the load at which the film is partially peeled is maximum, or a range in which twice the load at which brittle cracks occur without exhibiting ductility in the film is maximum.

At this time, the sample after the scratch test is observed by SEM/EDX, and the critical load can be obtained by measuring any short distance from the initial position of the scratch test to a position where brittle cracks occur without causing the film to exhibit ductility, a position where the film is partially peeled off, and a position where the substrate is exposed.

The critical load corresponds to adhesiveness between the film and the substrate, and the film of the biocompatible material of the present invention may have a critical load in the above range.

<<Appearance of Film after Scratch Test>>

Upon observing the sample after the scratch test by SEM/EDX, it is preferable that the film does not exhibit softness, that brittle cracks do not occur, that the film is not partially peeled off, and that the substrate is scraped in a ductile manner until being exposed.

<<Critical Shear Stress of Interface>>

From the torque required upon implantation of the implant screw, the shear stress generated on the screw surface can be calculated as follows:
Shear force $\tau_i$ generated on the surface of the screw can be calculated as in Equation 1. When the radius (1/2 of the diameter of the outer periphery) of the implant screw is r, the area where the screw is in contact with the alveolar bone is $A_i$, and the torque at the time of implantation is T, Equation 1 can be generally expressed.

$$\tau_i = \frac{T}{rA_i} \qquad \text{(Equation 1)}$$

Pressure and shear stress generated on the surface of the screw during implantation are concentrated near the tip of the ridge of the thread of the screw. Further, when the screw is implanted, high pressure and shear force are generated mainly in the area of the hard cortical bone. Since the average thickness of the cortical bone is about 1.0 to 1.5 mm, and since the pitch of the thread is 1 to 2 mm, the screw is screwed in a state where the shear force is concentrated on almost one round of the ridge of the thread. When the width of the tip of the ridge of the thread is h, the area $A_i$ of the tip of the ridge of the thread having the highest shear stress is expressed by Equation 2.

$$A_i = 2\pi rh \qquad \text{(Equation 2)}$$

Assuming that minimum implanting torque T for promoting bone formation is 40 Ncm (Non-Patent Document 3), the radius r of the implant screw is 2 mm, and the width of the tip of the ridge of the thread of the screw is 0.2 mm, calculation is performed by substituting these values into Equations 1 and 2 to obtain about 80 MPa as the shear stress $\tau_i$ generated on the surface. When the tip of the ridge of the screw is sharper, and the width of the ridge of the thread is 0.1 mm, the shear stress $\tau_i$ generated on the surface similarly is about 160 MPa. As described above, the shear stress becomes 80 to 160 MPa due to the shape of the tip of the ridge of thread, and the shear force increases as the tip of the ridge of the thread becomes sharper.

In order to prevent the film from peeling off, as seen in Equation 3, the adhesiveness at the interface between the film and the substrate (the shear stress $\tau_c$ at the interface) may be larger than the shear stress $\tau_i$ at the surface.

The shear stress $\tau_c$ at the interface between the film and the substrate for satisfying the implanting torque is preferably 80 MPa or more, preferably 160 MPa or more.

$$\tau_c > \tau_i \qquad \text{(Equation 3)}$$

<<Film Thickness>>

The thickness of the film of the biocompatible material of the present application is not particularly limited as long as the thickness has any one or two, or all of the characteristics i) to iii), the surface roughness, or a difference thereof, but for example, the average thickness may be 0.10 to 30 $\mu$m. In particular, the average thickness of the film corresponding to a contact portion with the bone and the vicinity thereof may be 0.10 to 30 $\mu$m, preferably 0.20 to 20 $\mu$m, more preferably 0.40 to 15 $\mu$m.

The content of calcium of the film may be preferably 0.8 to 35 % by weight, more preferably 5 to 30 % by weight, most preferably 15 to 25 % by weight.

The term "be free from $Mg_2Ca$" means that a peak based on $Mg_2Ca$ is not observed in X-ray diffraction analysis, and preferably that a peak based on $Mg_2Ca$ is not observed at a diffraction angle at which diffraction peaks generated from crystals of the substrate and $Mg_2Ca$ do not overlap each other (the respective diffraction peaks are separated by 1° or more in X-ray analysis by using a cobalt (Co) tube lamp), for example, a peak is not observed in a range of 36 to 37° in X-ray analysis using a Co tube lamp.

In one aspect of the present application, the film may have an amorphous portion.

Further, in one aspect of the present application, the film may be formed essentially of an amorphous material and preferably consists of an amorphous material.

The term "amorphous" means that no sharp peak is observed in X-ray diffraction analysis.

<Biocompatible Material>

The biocompatible material of the present invention may have a biocompatible substrate in addition to the film having the above characteristics.

The biocompatible substrate is not particularly limited as long as the biocompatible substrate has the "biocompatibility". Examples thereof may include, but are not limited thereto, pure titanium, a titanium alloy, a cobalt-chromium alloy, stainless steel, zirconia, and the like.

Examples of the biocompatible substrate may include, but are not limited thereto, alumina, calcium phosphate, magnesia, and the like.

The biocompatible substrate may preferably be pure titanium, a titanium alloy, or zirconia, more preferably ceramics, still more preferably zirconia.

Further, the biocompatible material of the present invention may have other layers in addition to the biocompatible substrate and the film described above. For example, one or more layers may be provided between the biocompatible substrate and the film. More, one or more layers may be provided on the upper portion of the above-mentioned film, that is, on the side opposite to the substrate.

The shape of the biocompatible material of the present invention is not particularly limited, but for example, the biocompatible material may be one selected from the group consisting of a columnar shape, a cylindrical shape, a truncated cone shape, a conical shape, a shape having a screw-shaped threaded portion in a part of the shape, a rectangular parallelepiped and a cube, a block shape such as a rectangular parallelepiped and a cube having a partially inclined surface, and a wedge shape.

The application field of the biocompatible material of the present invention is not particularly limited, but for example, the biocompatible material may be one selected from the group consisting of an artificial bone material, an intraosseous fixture material, a dental implant material, an orthodontic anchor screw material, a medullary nail material, and an interbody fixation material. Examples thereof may include, but are not limited to, an artificial bone, a pin, wire, a bolt, a screw, a washer, a medullary nail, and a vertebral body spacer.

<Method of Manufacturing Biocompatible Material>

The biocompatible material of the present invention can be manufactured, for example, by the following method.
The biocompatible material can be obtained by the method comprising the steps of:

(A) preparing a biocompatible substrate;
(B) preparing a sputtering target comprised of magnesium and optional calcium;
(C) cleaning a surface of the biocompatible substrate in vacuum; and
(D) sputtering by using the sputtering target and setting a temperature of the biocompatible substrate obtained in Step (C) at 130°C or lower, preferably 90°C or lower, more preferably 60°C or lower, to form the film comprised of magnesium and optional calcium on the biocompatible substrate.

Furthermore, as the "biocompatible substrate", those described above can be used.
The "film" is the same as that described above.
Step (A) is a step of preparing a biocompatible substrate. The above-described "biocompatible substrate" may be commercially purchased, or the commercially purchased substrate may be formed into a desired shape. The method may comprise a step of grinding and/or polishing the surface of the purchased product or the obtained shape. Furthermore, as the grinding method and the polishing method, conventional methods may be used.
Step (B) is a step of preparing a sputtering target comprised of magnesium and optional calcium.
A sputtering target may be prepared according to a film having a desired composition. For example, it may be manufactured by dissolving predetermined metal in a predetermined ratio.
Step (C) is a step of, appropriately adjusting a bias in vacuum, specifically in a vacuum chamber and causing argon ions or the like to collide with the surface of the substrate to remove impurities on the surface at an atomic level for cleaning before sputtering. Appropriate execution of the step (C) can lead stabilized adhesiveness of the film, and the activated surface of the substrate.
Step (D) is a step of using the sputtering target and setting a temperature of the biocompatible substrate at 130°C or lower, preferably 90°C or lower, more preferably 60°C or lower and sputtering, to form the film comprised of magnesium and optional calcium on the surface of the biocompatible substrate.
A magnetron sputtering device may be used as the sputtering device.
In the magnetron sputtering device, a strong magnet (magnetron) is disposed behind a target, and sputtered particles (metal particles) generated by causing argon ions to collide with the target can be efficiently deposited on a substrate using a magnetic field. Upon sputtering, the sputtering voltage, the bias of the substrate, the pressure in the device, and the temperature of a substrate material can be adjusted, to form a film at a constant film formation rate.
The metal film based on magnesium has a linear expansion coefficient three times larger or more than that of zirconium, titanium, or a titanium alloy as the substrate. Thus, when the sputtering temperature is high, the temperature difference from around room temperature at which the implant is used and the sputtering temperature becomes large, and thus, tensile stress (thermal stress) is generated at the interface on the film side, and the film is easily peeled off. Therefore, in order to prevent the film from being peeled off after the film formation and to prevent harmful stress from remaining at the time of implanting the implant, the temperature of the substrate in sputtering is controlled to a certain temperature or less, that is, 130°C or less, preferably 90°C or less, more preferably 60°C or less, so that a film having high adhesiveness can be formed.
The above temperature can be estimated by calculating thermal stress as follows: When the temperature is lowered from the sputtering temperature to room temperature, the stress (thermal stress) at the interface generated can be approximately expressed by Equation 4, wherein $\Delta T$: a temperature difference between a substrate temperature during sputtering Td and room temperature Tr, $\alpha_1$: an average linear expansion coefficient of the substrate between the temperatures Tr and Td, $\alpha_2$: an average linear expansion coefficient of the coating film between the temperatures Tr and Td, $E_1$: an average elastic modulus of the substrate between the temperatures Tr and Td, and $E_2$: an average elastic modulus of the film between the temperatures Tr and Td.

$$\sigma = \frac{E_1 E_2 (\alpha_1 - \alpha_2)\Delta T}{E_1(1 - \alpha_2 \Delta T) + E_2(1 - \alpha_1 \Delta T)} \qquad \text{(Equation 4)}$$

For example, assuming that the substrate is zirconia, and the coating film is pure magnesium, calculation is performed by applying the linear expansion coefficients as $8 \times 10^{-6}$ and $25 \times 10^{-6}$ and the elastic moduli as 210 GPa and 40 GPa, respectively.

Since the yield strength of pure magnesium is generally said to be about 90 to 100 MPa, the shear yield strength is about 50 MPa. In order to suppress the thermal stress to at least this value or less, the temperature difference may be 100 degrees or less. For example, when the operating temperature is 36°C as a body temperature, the film formation temperature may be 130°C or lower, and is preferably 90°C or lower, which is twice the safety factor or 60°C or lower in consideration of about three times the safety factor.

The manufacturing method of the present invention may comprise steps other than the steps (A) to (D) above. For example, as described above, after step (A) and before step (B), the method may comprise the step of forming the "biocompatible substrate" into a desired shape and the step of grinding and/or polishing the surface of the shape.

For example, when a layer is provided between the substrate and the film, the step of providing the layer may be provided after step (A) and before step (D).

Hereinafter, the present invention is specifically described with reference to, but is not limited only to, examples.

Examples

Examples in which the film does not comprise Ca and/or in which the film is not free of $Ca_2Mg$, are reference examples.

<Substrate Material>

As a substrate material assumed to be an implant, plate-shaped pure titanium A1 and zirconia A2 cut into $10 \times 10$ mm with a thickness of 3 mm and subjected to general grinding processing in a predetermined direction on one plane were used. Furthermore, in order to examine details of the characteristics of the film formed, a glass substrate AC1 having a thickness of 1.2 mm and $26 \times 76$ mm was also used.

Further, pure titanium B1 and zirconia B2 having a columnar shape ($\varphi$ 4 mm and the length of 10 mm) close to the shape of an actual dental root implant were used.

Furthermore, for any substrate, a part of the surface was masked in order to measure the thickness of the film formed.

<Sputtering Device and Sputtering Method>

As the sputtering device, a magnetron sputtering device was used.

As a sputtering target, pure magnesium, and four kinds of ingots manufactured by dissolving pure magnesium and pure calcium at a predetermined ratio were machined into a disk shape having a diameter of about 120 mm and used. The four kinds of ingots were those in which the amount of calcium was 0%, 10%, 20%, or 30%, and the remaining amount was magnesium. The % of the calcium amount is a ratio of the calcium weight when the total of the calcium weight and the magnesium weight is 100 % by weight and is represented by wt% as below.

Calcium wt% = Calcium Weight/(Magnesium Weight + Calcium Weight) $\times$ 100

The substrate was disposed on the stage of the sputtering device so as to face the sputtering target. Furthermore, the columnar substrate was disposed such that the columnar bottom surface was up and down, and the plate-shaped substrate was disposed such that the ground surface was the upper surface.

In the film formation process, first, the pressure was reduced to a predetermined value, the harmful gas in the chamber was removed, and then an argon gas was sealed.

By appropriately adjusting the voltage and the substrate bias required for discharge, the surfaces of the sputtering target and the substrate were subjected to ion cleaning to remove oxides and harmful compound layers on the surfaces, so that impurities that reduce adhesiveness at the interface between the substrate and the film are removed, and an active surface advantageous for bone formation is formed.

The temperature of the substrate was kept at room temperature, the pressure of argon was 1 to 10 mTorr, the sputtering voltage and the bias of the substrate were adjusted, and a film formation process (deposition) was performed for 12 hours.

The thickness of the obtained film was measured as follows: The thickness of the film was measured by actually measuring a gap distance by a stylus method, the gap distance being between a portion on the substrate where the film was formed and another portion where the film was not formed by performing the above-described masking.

The components of each film were measured with an energy dispersive X-ray analyzer (EDX).

The calcium wt% of the target, the kind of the substrate, the amount of Ca (wt%) according to the EDX results, and the film thickness ($\mu$m) obtained by the stylus method are shown in Table 1. Furthermore, the Ca amount (wt%) as a result of EDX for the columnar shape sample was considered to be equivalent to that of the plate-shaped sample, and thus the Ca amount (wt%) for the columnar shape was not measured.

Table 1. EDX results and the thickness of the film formed on Ti and ZrO$_2$ substrate

|  | Substrate | Ca amount (wt%) | Substrate with film | Thickness of film ($\mu$m) |
|---|---|---|---|---|
| 0%Ca | A1(Titanium) | 0 | A1-C0 | 5.107 |
|  | A2(zirconia) | 0 | A2-C0 | 5.282 |
|  | B1(Titanium) | - | B1-C0 | 2.662 |
|  | B2(zirconia) | - | B2-C0 | 2.520 |
| 10%Ca | A1(Titanium) | 9.50 | A1-C10 | 5.051 |
|  | A2(zirconia) | 9.28 | A2-C10 | 5.197 |
|  | B1(Titanium) | - | B1-C10 | - |
|  | B2 (zirconia) | - | B2-C10 | - |
| 20%Ca | A1(Titanium) | 18.15 | A1-C20 | 5.018 |
|  | A2 (zirconia) | 17.93 | A2-C20 | 4.857 |
|  | B1(Titanium) | - | B1-C20 | 2.455 |
|  | B2 (zirconia) | - | B2-C20 | 2.489 |
| 30%Ca | A1(Titanium) | 28.88 | A1-C30 | 5.042 |
|  | A2 (zirconia) | 28.25 | A2-C30 | 4.957 |
|  | B1(Titanium) | - | B1-C30 | 2.528 |
|  | B2 (zirconia) | - | B2-C30 | 2.603 |

Table 1 shows that the calcium wt% in the target and the calcium amount (wt%) of the formed film are almost the same. From this, it is found that there was no problem in the target and the sputtering. Although not illustrated, it has been confirmed that the film thickness increases in proportion to the time of the film formation process (deposition), and it has been found that the film thickness can be controlled by the film forming time. Also, the film thicknesses of "B1" and "B2" of "10% Ca" in Table 1 were not accurately measured but have been confirmed to be comparable to the film thicknesses of other "B1" and "B2".

<Arithmetic Mean Surface Height of Line Roughness>

For A1-C0, A1-C10, A1-C20, and A1-C30 formed as films by using titanium as a plate-shaped substrate, and for A2-C0, A2-C10, A2-C20, and A2-C30 formed as films by using zirconia as a plate-shaped substrate, the arithmetic mean heights Ra1 of the line roughness of the surfaces were measured.

Further, for A1-C0, A1-C10, A1-C20, and A1-C30 and for A2-C0, A2-C10, A2-C20, and A2-C30 formed as films by using zirconia as a plate-shaped substrate, the arithmetic mean heights Ra2 of the line roughness of the surfaces before film formation were measured.

More, for A1-C0, A1-C10, A1-C20, and A1-C30 and for A2-C0, A2-C10, A2-C20, and A2-C30 formed as films by using zirconia as a plate-shaped substrate, the films after film formation were immersed in a 5% aqueous hydrochloric acid solution for about 10 seconds to remove the film. The arithmetic mean heights Ra2' of the line roughness of the surfaces of the sample from which the films of the obtained samples were removed were measured. Furthermore, it was confirmed that Ra2 and Ra2' were substantially the same as described below.

The arithmetic mean heights of the line roughness were measured by moving the stylus in directions parallel and perpendicular to the grinding marks. The measurement was performed with a contact type surface roughness measurement device (SV-3000, manufactured by Mitutoyo Corporation) in accordance with JIS B0601: 2013. By using a stylus with a tip radius of 2 $\mu$m, the measurement was performed by contacting the sample with a static measurement force of 0.75 mN. Since the preliminary measurement resulted in 0.02 < Ra ($\mu$m) $\leq$ 0.1, the present measurement was performed with a reference length of 0.8 $\mu$m, an evaluation length of 4 mm, a cutoff $\lambda$c of 0.8 mm, and a $\lambda$s of 2.5 $\mu$m. Each sample was

measured 10 times, and the average value thereof was taken as Ra.

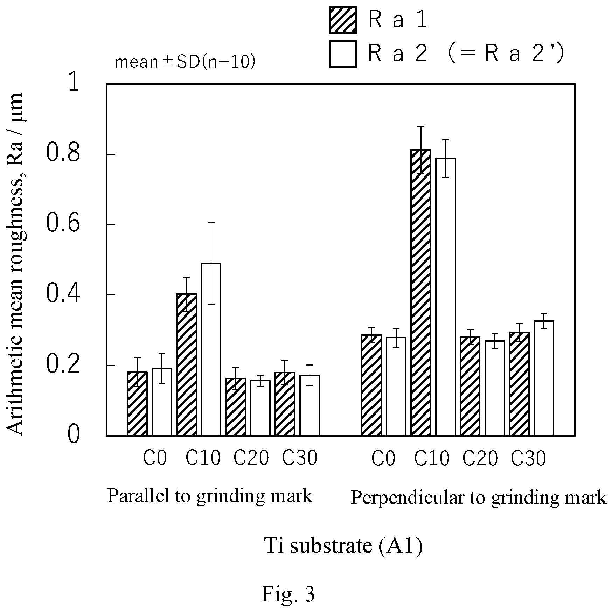

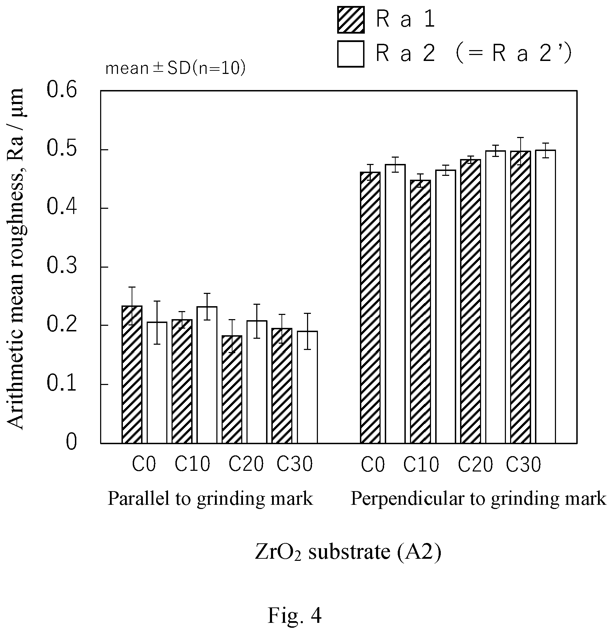

For each sample, the results of the arithmetic mean heights Ra1 of the line roughness of the surfaces having the films and the arithmetic mean heights Ra2 (= Ra2') of the line roughness of the surfaces without the films are illustrated in Figs. 3 and 4.

The arithmetic mean roughness of the surfaces without the films was measured for a) the measured values Ra2 before film formation and b) the measured values Ra2' of the surfaces removed after film formation. As a result, although not illustrated, it was confirmed that almost the same results were obtained. This result confirmed that b) the removal method after film formation was appropriate.

Fig. 3 is a graph illustrating measurement results of arithmetic mean surface roughness for the films A1-C0, A1-C10, A1-C20, and A1-C30 formed by using titanium as plate-shaped substrates. In the drawing, hatched bars show results for Ra1, and unhatched bars show results for Ra2.

Fig. 4 illustrates the results of the samples formed as films using "zirconia" instead of "titanium" as plate-shaped substrates in Fig. 3. Hatched bars show results for Ra1, and unhatched bars show results for Ra2 in a manner similar to Fig. 3.

Further, Table 2 is obtained by summarizing the values of Ra1 (hatched bars in Figs. 3 and 4) for each sample.

In addition, Table 3 is obtained by summarizing the difference between the values of Ra1 (hatched bars in Figs. 3 and 4) and the values of Ra2 (unhatched bars in Figs. 3 and 4) for each sample.

Table 2. Ra1 (nm) for each sample

| Substrate | Relationship of directions of grinding mark and measurement | Ca amount (wt%) | | | |
|---|---|---|---|---|---|
| | | 0 | 10 | 20 | 30 |
| Ti | Parallel | 181.5 | 402.1 | 163.5 | 180.4 |
| | Perpendicular | 286.3 | 811.9 | 280.4 | 293.8 |
| ZrO$_2$ | Parallel | 233.5 | 209.8 | 182.2 | 194.7 |
| | Perpendicular | 460.9 | 446.9 | 482.5 | 497.2 |

Table 3. The difference (Ra1-Ra2) (nm) between Ra1 and Ra2 for each sample

| Substrate | Relationship of directions of grinding mark and measurement | Ca amount (wt%) | | | |
|---|---|---|---|---|---|
| | | 0 | 10 | 20 | 30 |
| Ti | Parallel | -10.5 | -87.2 | 6.0 | 8.2 |
| | Perpendicular | 7.5 | 24.9 | 11.5 | -32.1 |
| ZrO$_2$ | Parallel | 28.4 | -22.4 | -25.4 | 4.7 |
| | Perpendicular | -13.3 | -17.7 | -15.5 | -1.6 |

<Arithmetic Mean Surface Height of Surface Roughness>

For films A1-C0, A1-C10, A1-C20, and A1-C30 formed by using titanium as a plate-shaped substrate, and for films A2-C0, A2-C10, A2-C20, and A2-C30 formed by using zirconia as a plate-shaped substrate, the arithmetic mean heights Sa1 of the surface roughness of the surfaces were measured.

Further, for films A1-C0, A1-C10, A1-C20, and A1-C30 and for films A2-C0, A2-C10, A2-C20, and A2-C30 formed by zirconia using as a plate-shaped substrate, the arithmetic mean heights Sa2 of the surface roughness of the surfaces before film formation were measured.

More, for films A1-C0, A1-C10, A1-C20, and A1-C30 and for films A2-C0, A2-C10, A2-C20, and A2-C30 formed by using zirconia as a plate-shaped substrate, the films after film formation were immersed in a 5% aqueous hydrochloric acid solution for about 10 seconds, and the films were removed. The arithmetic mean heights Sa2' of the surface roughness of the surfaces of the sample, from which the films of the obtained samples were removed, were measured. Furthermore, it was confirmed that Sa2 and Sa2' were substantially the same as described below.

The arithmetic mean heights of the surface roughness were measured by deflecting the measurement probe in directions perpendicular to the grinding marks. The measurement was performed with a laser probe type noncontact three-dimensional measuring device (NH-3 SP, manufactured by Mitaka Kohki Co., Ltd.) in accordance with ISO 25178. A laser having a probe diameter of 1 $\mu$m was used to measure a range of 100 $\mu$m $\times$ 100 $\mu$m at a measurement pitch of 1 $\mu$m. The measurement of the arithmetic mean height of the surface roughness was carried out by using analysis software TalyMapGold (Version 7, manufactured by AMETEK Taylor Hobson). The arithmetic mean height was calculated without using a filter after subjecting a calibration surface leveled by a least squares method to shape removal by a quadratic polynomial and separating wavelength components of a contour curve.

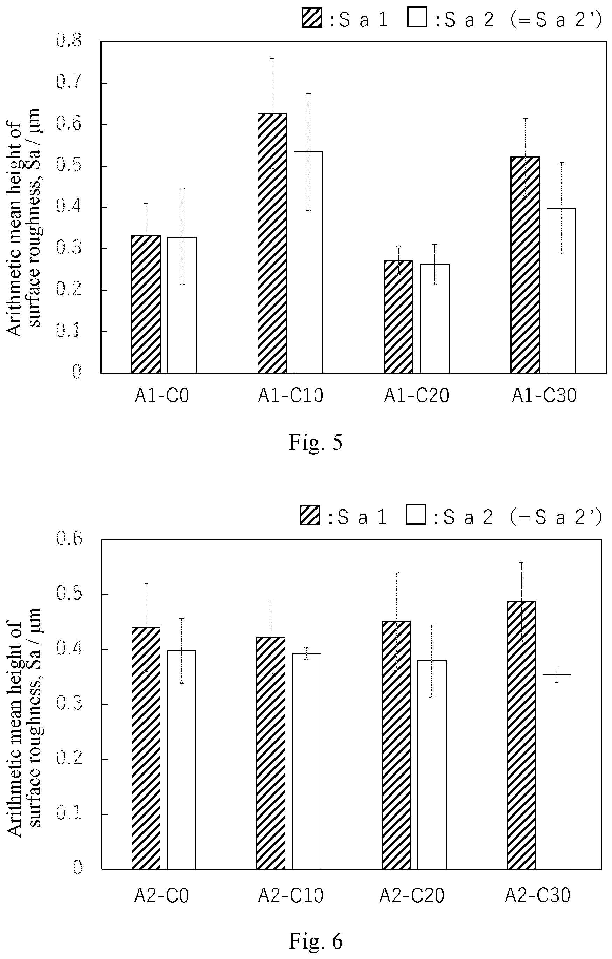

For each sample, the results of the arithmetic mean heights Sa1 of the surface roughness of the surfaces having the films and the arithmetic mean heights Sa2 (= Sa2') of the surface roughness of the surfaces without the films are illustrated in Figs. 5 and 6.

The arithmetic mean roughness of the surfaces without the films was measured for a) the measured values Sa2 before film formation and b) the measured values Sa2' of the surfaces removed after film formation. As a result, although not illustrated, it was confirmed that almost the same results were obtained. From this result, it was confirmed that b) the removal method after film formation was appropriate.

Fig. 5 is a graph illustrating measurement results of arithmetic mean surface roughness for films A1-C0, A1-C10, A1-C20, and A1-C30 formed by using titanium as plate-shaped substrates. In the drawing, hatched bars show results for Sa1, and unhatched bars show results for Sa2.

Further, Fig. 6 illustrates the results of the samples formed as films using "zirconia" instead of "titanium" as plate-shaped substrates in Fig. 5. Hatched bars show results for Sa1, and unhatched bars show results for Sa2 in a manner similar to Fig. 5.

More, Table 4 is obtained by summarizing the values of Sa1 (hatched bars in Figs. 5 and 6) for each sample.

Further, Table 5 is obtained by summarizing the difference between the values of Sa1 (hatched bars in Figs. 5 and 6) and the values of Sa2 (unhatched bars in Figs. 5 and 6) for each sample.

Table 4. Sa1 (nm) for each sample

| Substrate | Ca amount (wt%) | | | |
|---|---|---|---|---|
| | 0 | 10 | 20 | 30 |
| Ti | 331.4 | 626.7 | 271.6 | 521.3 |
| $ZrO_2$ | 440.3 | 422.3 | 451.8 | 487.3 |

Table 5. The difference between Sa1 and Sa2 (Sa1-Sa2) (nm) for each sample

| Substrate | Ca amount (wt%) | | | |
|---|---|---|---|---|
| | 0 | 10 | 20 | 30 |
| Ti | 2.8 | 92.8 | 9.6 | 124.5 |
| $ZrO_2$ | 42.7 | 29.5 | 72.5 | 133.4 |

Figs. 3, 4, 5, and 6, and Tables 2, 3, 4, and 5 show the following things:
In the implantation of, for example, dental root implant materials, it is important to have a relatively smooth surface roughness in reducing the burden on the patient and in appropriate implantation and clamping, but this depends on the maximum value of the arithmetic mean surface height of the line roughness of the sample and on the maximum value of the arithmetic mean surface height of the surface roughness. Since the present examples from Tables 2 and 4 show that the maximum value of the arithmetic mean surface heights of the line roughness or the surface roughness is 1 $\mu$m or less, the sample obtained in the present example can be smoothly implanted. In the present example, a substrate subjected to a general grinding process is used, but as described below in a comparative example, application of a smoother substrate can provide smoother the surface roughness. Such a substrate makes it possible to shorten a period until secondary fixation after implantation in a screw (bone screw or anchor screw), which is used on the premise of removal thereof, to suppress physical engagement due to unevenness of the surface of the substrate at the time of removal, and to smoothly perform removal.

Further, the formation of the film by the example using sputtering can relatively minimize the difference value (Ra1 - Ra2) (nm) between Ra1 and Ra2 and the difference value (Sa1 - Sa2) (nm) between Sa1 and Sa2 of each sample. Since the maximum value of the surface roughness of the film is 30 nm or less as described below in the comparative example, the surface roughness is hardly increased by forming the film.

More, reduction of the difference indicates that the film is not locally formed but is uniformly formed all over the surface of the substrate. It can be expected that the effect of the film is stably exhibited throughout the substrate.

<Scanning Electron Microscope Image>

For the sample obtained, the cross-sectional direction of the film and the surface having the film were confirmed with a scanning electron microscope (SEM).

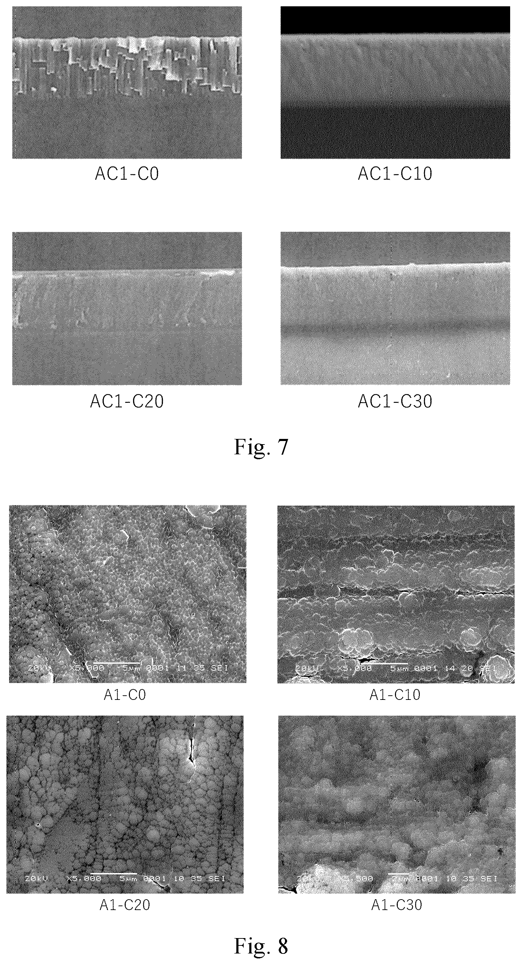

Fig. 7 illustrates scanning electron microscope images (SEM images) of the films in the cross-sectional direction for films AC1-C0, AC1-C10, AC1-C20, and AC1-C30 formed by using a glass substrate.

Fig. 8 illustrates scanning electron microscope images of the surfaces with the films for films A1-C0, A1-C10, A1-C20, and A1-C30 formed by using titanium as plate-shaped substrates.

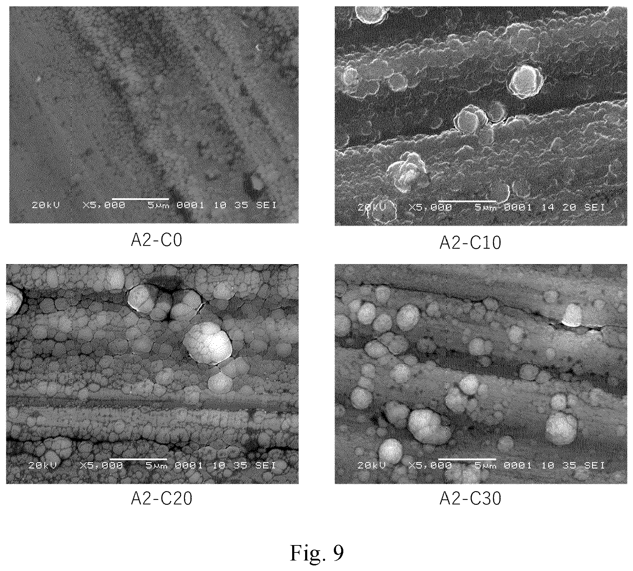

Fig. 9 illustrates scanning electron microscope images of surfaces with the films for films A2-C0, A2-C10, A2-C20, and A2-C30 formed by using zirconia as plate-shaped substrates.

Fig. 7 shows that in the case of calcium 0% (AC1-C0), columnar crystals of magnesium are densely arranged from the interface of the substrate with orientation in the vertical direction to the interface. However, in the film of 10% calcium (AC1-C10), columnar orientation is not observed, and it is found that fine particles are dispersed in a disordered direction. Also, it is found that, at 20% calcium (AC1-C20) and 30% calcium (AC1-C30), no fine particles were observed, and thus the surface was very smooth.

Further, from the appearance of the upper surfaces of Figs. 8 and 9, in the case of using only magnesium (A1-C0 and A2-C0), the surface is relatively smooth, but a hexagonal columnar cross section is partially seen. This corresponds to the (0001) plane of the hexagonal close-packed structure (HCP) which is a magnesium crystal lattice, and it has been found that the crystal was oriented in this direction. It is found that fine particles having no directionality are dispersed in 10% calcium (A1-C10 and A2-C10). Also, it is found that 20% calcium (A1-C20 and A2-C20) and 30% calcium (A1-C30 and A2-C30) each has a smooth surface as in the cross section.

<X-Ray Diffraction Analysis>

X-ray diffraction analysis was carried out for the samples obtained, films AC1-C0, AC1-C10, AC1-C20, and AC1-C30 formed by using a glass substrate, films A1-C0, A1-C10, A1-C20, and A1-C30 formed by using titanium as a plate-shaped substrate, and films A2-C0, A2-C10, A2-C20, and A2-C30 formed by using zirconia as a plate-shaped substrate.

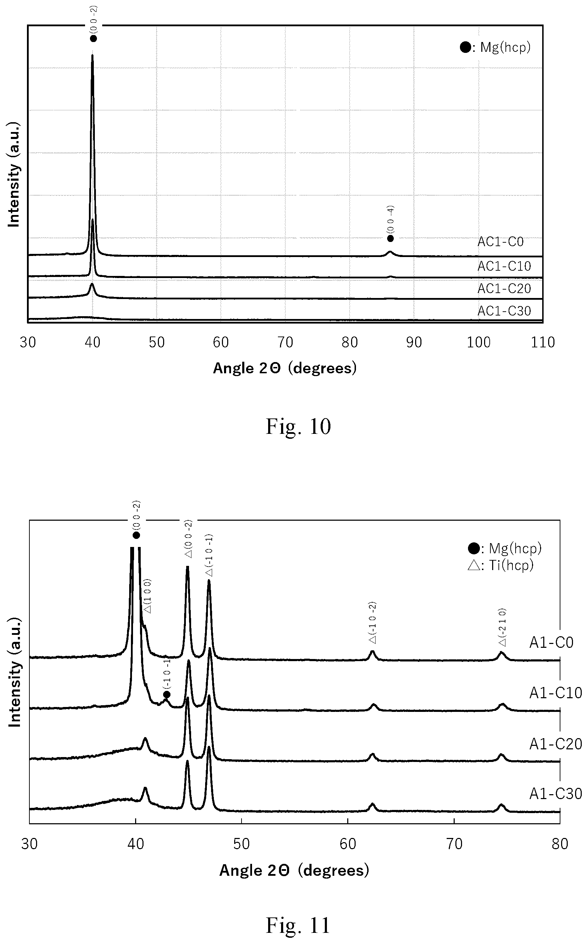

Specifically, X-ray diffraction analysis was carried out by using an X-ray diffractometer (D8 ADVANCE, manufactured by Bruker Corporation) under the conditions of a detector: a two-dimensional detector, tube lamp: Co, tube lamp voltage: 30 kV, tube lamp current: 40 mA, slit: $\Phi$ 1.0 mm, and collimator: $\Phi$ 1.0 mm. Results are shown in Figs. 10, 11, and 12.

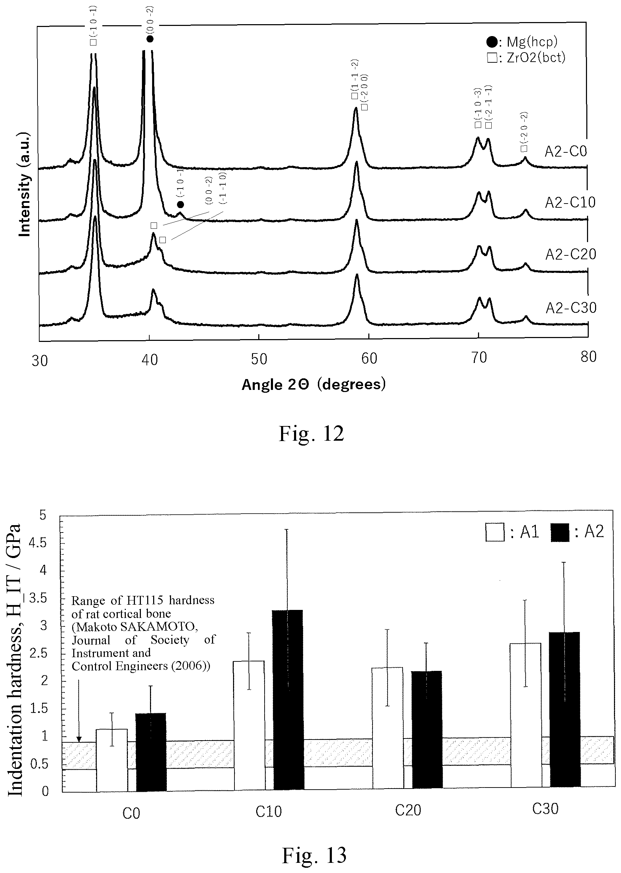

Peaks are observed near $2\theta = 40$ degrees in any film in Fig. 10. In the case of 0% calcium (AC1-C0), the diffraction intensity is very high and sharp, and the peak decreases as the calcium in magnesium increases. In the case of 30% (AC1-C30), the peak becomes broader and cannot be clearly seen. In 20% calcium (AC1-C20), a low peak is observed, but the position of the peak is shifted to the lower angle side to become broader, and it is found that the lattice interval is widened and distorted. From Figs. 11 and 12, it is found that the same results are obtained when any film is formed on the plate-shaped substrate using titanium and the plate-shaped substrate using zirconia.

These results show that calcium up to 10% has crystallinity, but the film becomes substantially amorphous when calcium exceeds 20%, from the results of X-ray diffraction (Figs. 10, 11, and 12) and SEM images (Figs. 7, 8, and 9).

The reason why the intensity of the (00-2) peak near 40 degrees is remarkably high in the film of 0% calcium is considered to be due to the orientation of crystals also observed in the appearance of the SEM image (Figs. 7, 8, and 9). In the film of 10% calcium of the plate-shaped substrate using titanium and the plate-shaped substrate using zirconia, not only the peak of (00-2) but also the peak of (-10-1) are observed, and therefore it is considered that the plate-shaped substrate using titanium and the plate-shaped substrate using zirconia are fine crystals having a random orientation.

It has been found that, as the proportion (%) of calcium increases, the dense columnar structure changes into fine crystals and amorphous, and this also appears in the crystallite size illustrated in Table 6. It is found that, as the proportion of calcium increases, the crystallite size decreases, and that the structure becomes more amorphous. The crystallite size was calculated using the Scherrer method from the integral width of the peak near $2\theta = 40$ degrees (00-2) where a film is formed on the glass substrate.

This structural change is also shown in the characteristics of the arithmetic mean surface roughness described above. That is, the surface roughness is small and smooth in any sample.

Table 6. Size of crystallite (nm) for each sample

| Substrate | Ca amount (wt%) | | | |
|---|---|---|---|---|
| | 0 | 10 | 20 | 30 |
| Glass | 152.3 | 205.6 | 52.9 | 16.5 |

<Hardness Test>

For films A1-C0, A1-C10, A1-C20, and A1-C30 formed by using titanium as a plate-shaped substrate, and for films A2-C0, A2-C10, A2-C20, and A2-C30 formed by using zirconia as a plate-shaped substrate, a hardness test was carried out by a nanoindentation method.

A hardness test by a nanoindentation method was carried out by using an apparatus (an ultra-micro indentation hardness tester "ENT-1100A" of ELIONIX INC.) under conditions: Berkovich indenter as an indenter; an indentation load of 20 mN; and retention time of 0 second.

The results of the hardness test are shown in Fig. 13.

Fig. 13 showed that all films had a higher hardness than hardness of bones of 0.4 to 0.9 GPa and had sufficient "abrasion resistance" to bones.

<Adhesiveness of Film by Scratch Test>

For films A1-C0, A1-C10, A1-C20, and A1-C30 formed by using titanium as a plate-shaped substrate, and for films A2-C0, A2-C10, A2-C20, and A2-C30 formed by using zirconia as a plate-shaped substrate, a scratch test was carried out.

A scratch tester CSR 1000 (manufactured by RHESCA CO., LTD.) was used as a scratch tester, and a load was applied in a range of 0 to 8 kg by weight by using a Rockwell indenter having a diameter of 0.8 mm. The sample after the scratch test was observed with SEM/EDX, and the critical load was obtained by measuring the distance from the initial position of the scratch test to the position where the substrate was exposed.

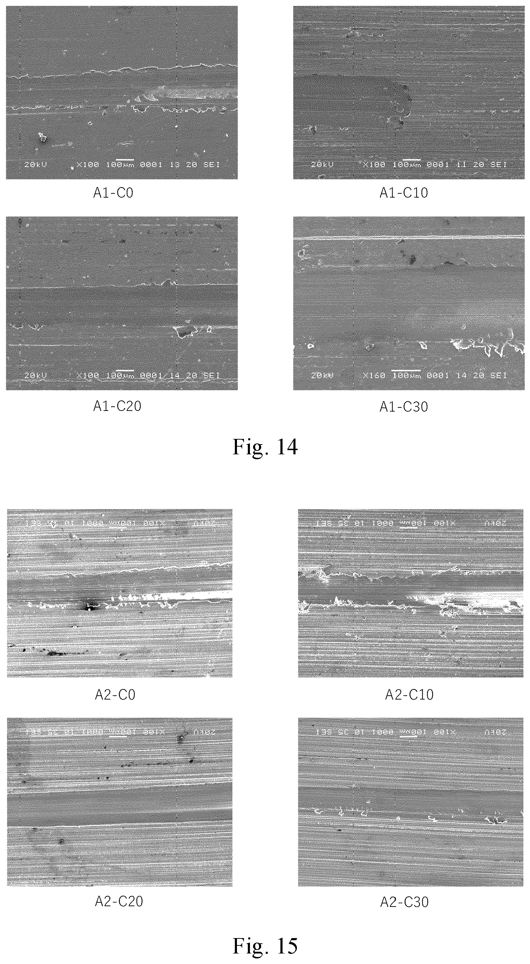

Fig. 14 is SEM images of appearances near critical loads after scratch tests in a direction parallel to grinding marks for films A1-C0, A1-C10, A1-C20, and A1-C30 formed by using titanium as plate-shaped substrates. Fig. 15 is SEM images of appearances near critical loads after scratch tests in a direction parallel to grinding marks for films A2-C0, A2-C10, A2-C20, and A2-C30 formed by using zirconia as plate-shaped substrates.

Figs. 14 and 15 show that in all the films, no partial peeling of the film was observed, and that the "adhesiveness" was good, indicating that the abrasion resistance of the film can be sufficiently exhibited when a force is applied to the surface.

Further, the results of Figs. 14 and 15 show that, in all the films tested, no flaky fractures or cracks during deformation are observed, and that the films exhibit sufficient "ductility" and are not brittle.

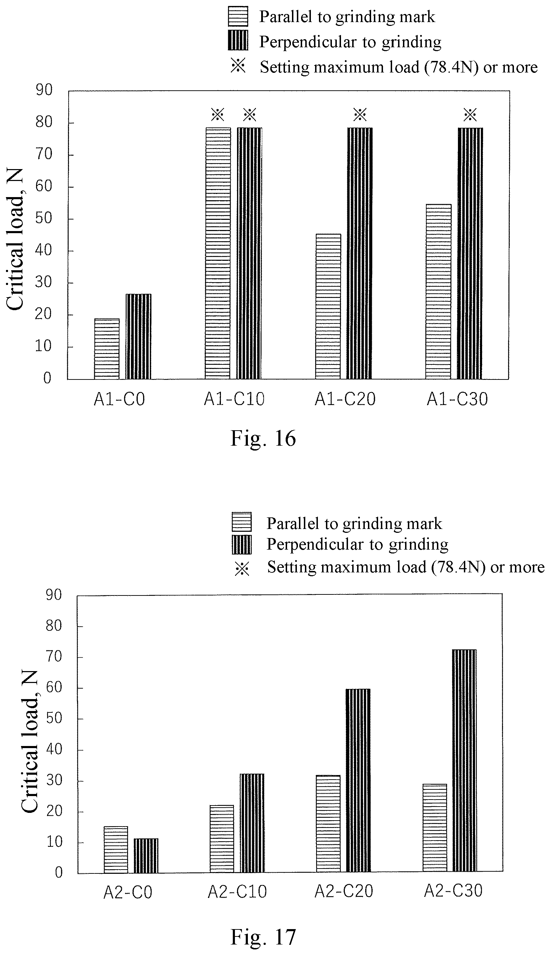

Fig. 16 illustrates measurement results for finding loads when an indenter almost reaches substrates, that is, critical loads of films in scratch tests in a direction parallel or perpendicular to grinding marks of films A1-C0, A1-C10, A1-C20, and A1-C30 formed by using titanium as plate-shaped substrates.

Fig. 17 illustrates measurement results for finding loads when the indenter almost reaches the substrates, that is, critical loads of the films in scratch tests in a direction parallel or perpendicular to grinding marks of films A2-C0, A2-C10, A2-C20, and A2-C30 formed by using zirconia as plate-shaped substrates. The critical load also indicates the degree of adhesiveness of the film.

Figs. 16 and 17 show that:

Among films A1-C0, A1-C10, A1-C20, and A1-C30 formed by using titanium as the plate-shaped substrate and films A2-C0, A2-C10, A2-C20, and A2-C30 formed by using zirconia as the plate-shaped substrate, A1-C10 in the scratch test in the direction parallel to the grinding mark and A1-C10, A1-C20, and A1-C30 in the scratch test in the direction perpendicular to the grinding mark have critical loads of 78.4 N or more, indicating that A1-C10, A1-C20, and A1-C30 are excellent in adhesiveness.

Even in case of A2-C0 having the smallest critical load, the critical load is about 10 N and satisfies 10 N or more. The critical load of about 10 N corresponds to an implanting torque of 150 Ncm that can be implanted without peeling the film in an implant screw having a screw radius of 2 mm and the width of the tip of the ridge of the thread of 0.2 mm. The 150 Ncm has a strength of a safety factor of about 4 times the implanting torque of 35 to 45 Ncm required for the implant, indicating that the films according to the present examples have desired adhesiveness.

<Critical Shear Stress of Interface>

The critical shear stress of the interface can be obtained by using the result of the scratch test.

In the scratch test, the load W is applied to the surface of the film through an indenter, and the tip of the indenter is dragged (squeezed) in parallel with the film, so that the length is measured until the film is peeled off, and the surface of the substrate is exposed. Since the load of the indenter is set to be proportional to the dragging length, the critical load Wc when the film is peeled off can be known from this length. At this time, the brittleness and ductility of the film are also found by observing the appearance of breakage or deformation of the film. These values are obtained using the commonly used Benjamine-Weaver's equation.

The load when the film is broken is defined as the critical load Wc, the radius of the tip of the diamond indenter is defined as R, and the hardness of the Brinel of the substrate is defined as $H_E$, and thus the shear stress $\tau_c$ (critical shear stress) at the interface at this time is expressed by the following Equation 5.

$$\tau_c = \sqrt{\frac{W_c}{\pi R^2 H_B - W_c} \cdot H_B} \qquad \text{(Equation 5)}$$

A tip R of the diamond indenter was set to 0.8 mm, the Brinell hardness of the titanium substrate and the zirconia substrate was set to 2.3 GPa and 12.0 GPa, respectively, and the critical load was calculated using the values of the scratch test in Figs. 16 and 17. Since the Brinell hardness of 10 GPa or more is not in the conversion table, hardness of zirconia takes the value of the hardness of Vickers using diamond as an indenter, which shows substantially the same value as Brinell. Further, for the critical load, a result of a scratch test in a direction with a smaller value (direction parallel to the grinding mark) was used.

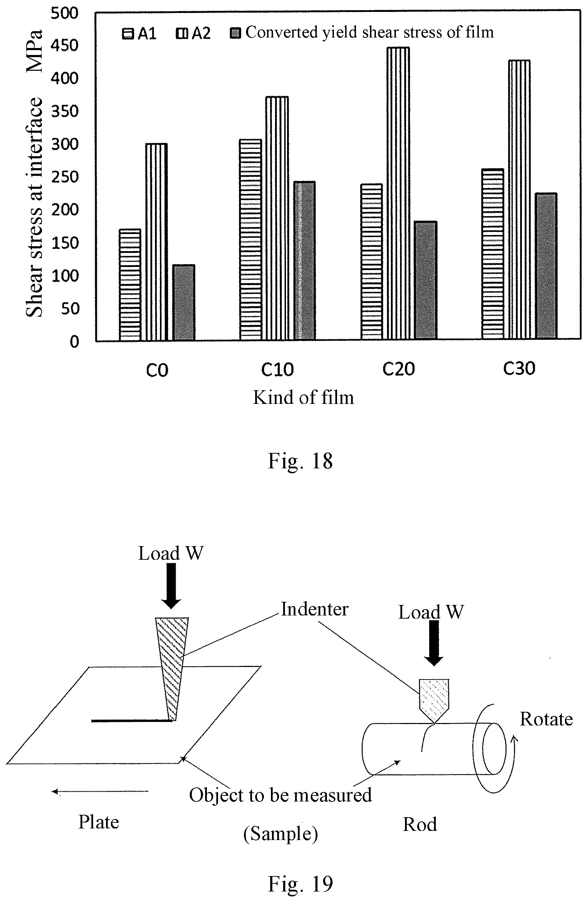

As shown in Fig. 18, in the combinations of all the films A1-C0 to A1-C3 and A2-C0 to A2-C3 and the substrate, the shear stress $\tau_c$ at the interface was significantly more than 80 MPa and more than 160 MPa. As a result, it has been found that the shear stress at the interface of the film produced in the present invention satisfies Equation 3, that the adhesiveness is sufficiently stronger than the shear stress generated at the outer peripheral portion at the time of implanting, and that the film is not peeled off at the time of implanting.

Fig. 18 illustrates yield shear stresses of A1-C0 to A1-C30 and A2-C0 to A2-C30 converted from the hardness of the films A1-C0 to A1-C30 and A2-C0 to A2-C30. Further, a method of calculating the yield shear stresses can be seen in, for example, G.E. Dieter: Mechanical metallurgy, McGraw-Hill, 1988.

The yield stress of the film is smaller than the shear stress at the interface of the film, and it is found that, when a shear force is applied to the surface, the film is deformed in a ductile manner prior to the fracture at interface and then does not peel off from the interface of the film. This was apparent in the appearance of the scratch test in Figs. 14 and 15.

In the scratch test, when the substrate has a cylindrical shape like a screw, as illustrated in Fig. 19, the indenter is set on the surface in a direction perpendicular to the axis of the cylinder, and the indenter is dragged along the circumferential direction by rotating the cylinder about the axis in a state where the load W is applied in the radial direction of the cylinder, so that the same measurement as in the case of the flat surface can be performed, and the critical load can be obtained. However, in this measurement, since the substrate is cylindrical, the value of $W_c$ is corrected according to Hertz's theoretical formula (see K. L. Johnson: Contact mechanics, Cambridge university press, 1985). For example, $W_c$ takes about 1.96 times larger value for a Φ4 mm cylinder and about 1.74 times larger value for a Φ5 mm cylinder.

The results of <Hardness Test> and <Scratch Test> show that the films according to the present invention formed on the implant screw have sufficient abrasion resistance, adhesiveness, and ductility, which means the films have excellent characteristics that have not been seen before as a film of the implant screw.

<In Vitro Biological Reaction Test Using Pseudo Body Fluid>

For films A1-C0, A1-C10, A1-C20, and A1-C30 formed as by using titanium as a plate-shaped substrate, and for films A2-C0, A2-C10, A2-C20, and A2-C30 formed by using zirconia as a plate-shaped substrate, an in vitro biological reaction test using a pseudo body fluid was carried out.

As the pseudo body fluid, a Hank's balanced salt solution (HBSS (+) solution) containing calcium and magnesium was used.

As the pseudo body fluid immersion test, each sample was immersed in 400 ml of the pseudo body fluid, and held in a thermostatic bath at 37°C for one week, and then the sample was taken out from the solution.

Further, in order to bring the equilibrium pH in the solution close to the in vivo environment, the solution amount of HBSS (+) was adjusted so that the equilibrium pH after elution of the sputtering film was 8.0 or less. For comparison, each substrate without a film was also tested.

The result of observing the state of the surface on which the film was formed with time showed that almost all of the formed films were eluted into the liquid, and then new films were formed on the surface.

Although not illustrated, in the substrates of titanium and zirconia on which sputtering films were not formed, a new film was not formed in HBSS, but in the substrates of 0% calcium (pure magnesium) to 30% calcium formed by sputtering, the formation of new films was recognized. In addition, it was observed that the film including 10% or more of calcium had a large adhesion amount of a new film.

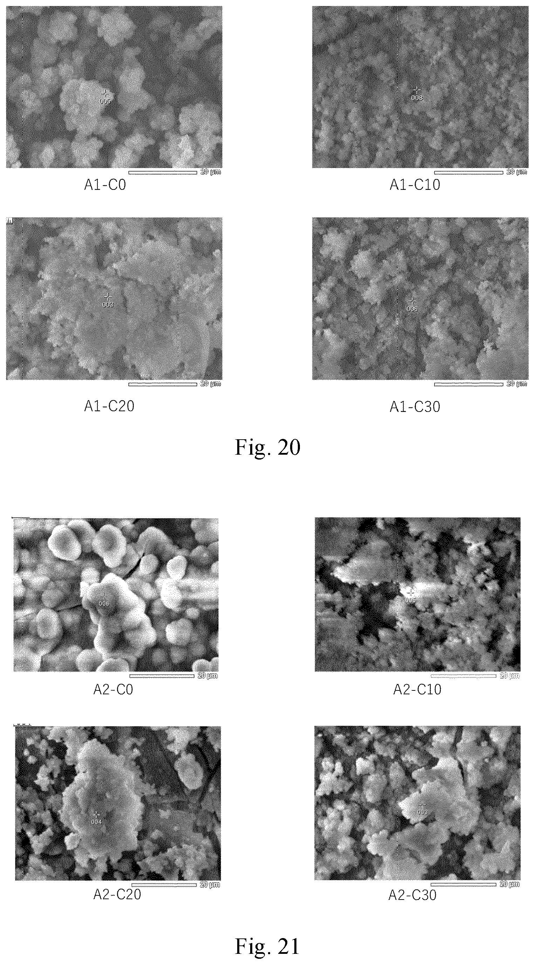

Fig. 20 illustrates SEM images of films newly formed on the substrates by immersing films A1-C0, A1-C10, A1-C20, and A1-C30 formed by using titanium as plate-shaped substrates in HBSS for one week.

Fig. 21 illustrates SEM images of films newly formed on the substrates by immersing films A2-C0, A2-C10, A2-C20, and A2-C30 formed by using zirconia as plate-shaped substrates in HBSS for one week.

Further, the results of component analysis performed by fluorescent X-rays around the center (specifically, the position of "+" in the SEM image) in the SEM image are shown in Table 7.

Table 7. The results of component analysis by fluorescent X-rays

| Sample | Element (% by mass) | | | | | |
|--------|------|-------|------|------|-------|-------|
|        | C    | O     | Na   | Mg   | P     | Ca    |
| A1-C0  | 13.84 | 54.35 | 0.36 | 0.87 | 11.15 | 19.44 |
| A1-C10 | 8.30  | 44.94 | 0.26 | 0.96 | 15.72 | 29.81 |
| A1-C20 | 14.11 | 49.82 | 0.49 | 0.83 | 12.52 | 22.23 |
| A1-C30 | 10.77 | 43.54 | 0.28 | 1.01 | 15.00 | 29.41 |
| A2-C0  | 10.71 | 50.82 | 0.27 | 1.53 | 14.18 | 22.48 |
| A2-C10 | 8.44  | 51.46 | 0.28 | 0.82 | 13.63 | 25.37 |
| A2-C20 | 8.74  | 44.74 | 0.30 | 1.59 | 16.16 | 28.48 |
| A2-C30 | 9.61  | 55.58 | 0.36 | 0.88 | 12.68 | 20.90 |

In Table 7, the ratio of calcium to phosphorus, which is an index of the presence of apatite, showed a value of 1.5 to 2.0 in all the sputtering films, suggesting that there was a high possibility that apatite was formed as a component.

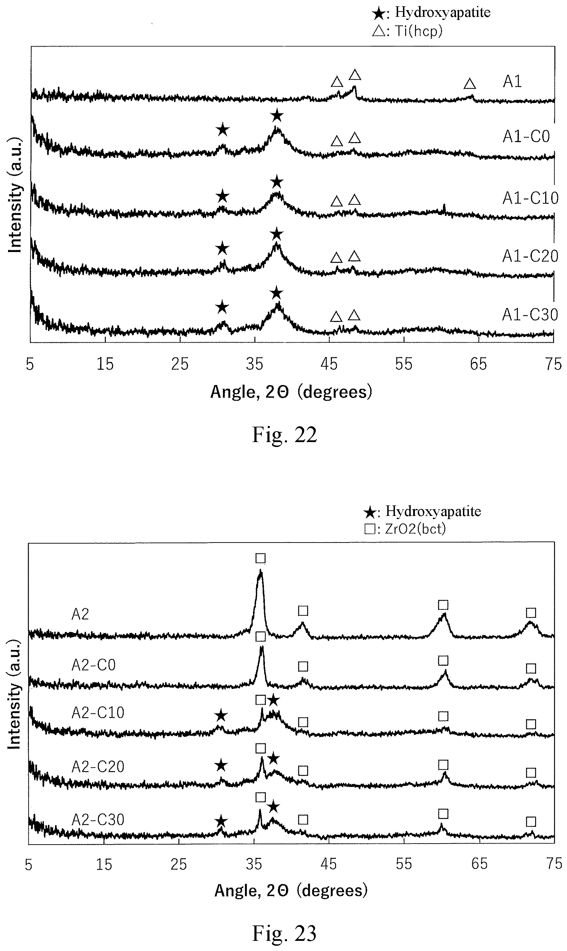

Further, in order to analyze the structure of the film, X-ray diffraction (XRD) analysis was carried out by a thin film method in which only a detector was scanned while an X-ray incident angle was fixed. Results are shown in Figs. 22 and 23.

On most substrates sputtered, peaks appeared at diffraction angles representing the characteristics of apatite on both the titanium substrate and the zirconia substrate. In A2-C0, the peak of apatite was not confirmed, but apatite was present on the surface as shown in Fig. 21 and Table 7, suggesting that the amount of apatite formed on A2-C0 is smaller than that of the sample having other sputtering films.

As described above, from component analysis and structural analysis, it was able to be confirmed that apatite was formed on all the substrates having the sputtering film. Further, it was able to be confirmed that the amount of apatite to be formed was increased by containing calcium in the sputtering film.

(Comparative Example)

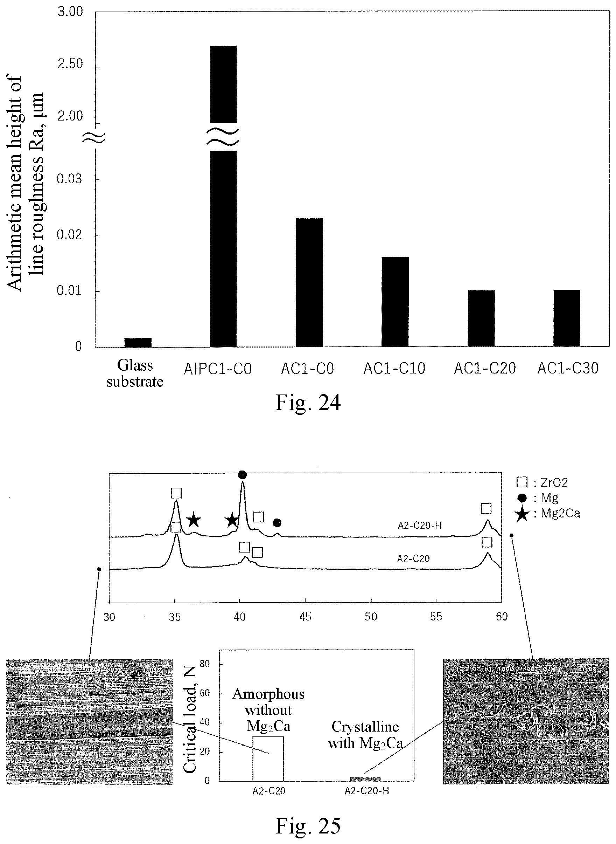

Fig. 24 illustrates comparison of an arithmetic mean height Ra1 of line roughness between a sample AIPC1-C0 formed as a film by using pure magnesium by arc ion plating using a glass substrate having a smooth surface, in order to evaluate the surface roughness of only the film and films AC1-C0, AC1-C10, AC1-C20, and AC1-C30 formed by using pure magnesium by sputtering using a glass substrate. When the surface roughness of the sample in which a film is formed using pure magnesium by arc ion plating is compared with that of the sample in which a film is formed using pure magnesium by sputtering, it is found that when a film is formed using pure magnesium by arc ion plating on the glass substrate before film formation, Ra is increased by 2.5 $\mu$m or more. Meanwhile, it is found that when a film is formed using pure magnesium by sputtering, Ra increases only by 0.03 $\mu$m or less. More, it is found that the increase in roughness during film formation is further suppressed by containing calcium.

Fig. 25 illustrates a comparison of results of an XRD and a scratch test between the film A2-C20 formed by using zirconia as a plate-shaped substrate and A2-C20-H obtained by subjecting the film to a heat treatment. The heat treatment was maintained for one hour at 200°C in vacuum and then the furnace was cooled. The XRD results show that A2-C20 has an amorphous which is a structure free from $Mg_2Ca$, and A2-C20-H has a structure in which magnesium crystals and $Mg_2Ca$ are mixed. A2-C20 having an amorphous which is the structure free from $Mg_2Ca$ had a critical load of about 30 N, while A2-C20-H having a structure in which magnesium crystals and $Mg_2Ca$ are mixed had a critical load of about 2.7 N. Further, cracks and partial peeling were not observed in the photograph of A2-C20 after the scratch test. Meanwhile, a lot of cracks or partial peeling was observed in the photograph of A2-C20-H after the scratch test.

These show that the amorphous which is a structure free from $Mg_2Ca$ has non-brittle characteristics and exhibits a high critical load. Meanwhile, it is found that when $Mg_2Ca$ is present and brittle, the critical load is significantly low.

Table 8 shows a comparison of the adhesion strength between the film in the related art and the film of the present application.

The adhesion strength of the calcium titanate film disclosed in Patent Document 2 is 4.9 MPa, and the adhesion strength of the calcium phosphate coating film disclosed in Non-Patent Document 2 is 80 MPa. These are tensile strengths obtained in pin tensile tests using adhesives.

The tensile strength of the film of the present application was measured by a similar pin tensile test, but the tensile strength of the adhesive was too strong to be measured as the adhesive was broken earlier. For a sample of which the adhesion strength cannot be measured by a pin tensile test, a scratch test or the like capable of measuring stronger adhesion strength is generally used. According to the in-vivo implantable material field development guidelines 2008 issued by the Ministry of Economy, Trade and Industry, it is desirable that both the tensile strength and the shear strength of the film be 20 MPa or more. Therefore, the shear stress calculated in the present example and the tensile strength in the prior art can be compared as adhesion strength at the same level. Therefore, it is found that, even when the adhesion strength of A2-C20 is 170 MPa, which is the lowest among those calculated in the present example, the adhesion strength is an extremely high value as compared with the related art.

Table 8.

| Name | Source of reference | Measuring test | Adhesive strength |
|---|---|---|---|
| Film for Artificial hip prosthesis | Development guidelines 2008 | Tensile test, Shearing test | 20 MPa or more |
| Calcium titanate film | Patent Document 2 | Tensile test | 4.9 MPa |
| Calcium phosphate film | Non-Patent Document 2 | Tensile test | 78 MPa |
| A2-C20 | Present application | Tensile test | Undetermined |
| A2-C20 | Present application | Scratch test (converted into shearing stress) | 170 MPa |

**Claims**

1. A biocompatible material having a film consisting of magnesium and calcium,
wherein
a content of calcium in the film is more than 0 % by weight and 40 % by weight or less where a total weight of magnesium and calcium is 100 % by weight, and the film is free from $Mg_2Ca$.

2. The biocompatible material according to claim 1,
wherein the film has an amorphous portion.

3. The biocompatible material according to claim 1 or 2, wherein the film has any one, two, or all of following characteristics i) to iii):

   i) hardness obtained by an indentation test is 0.4 GPa or more;
   ii) a relationship Wc/t between a critical load Wc (N) and a film thickness t ($\mu$m) is 1 N/$\mu$m or more; and
   iii) a critical shear stress at an interface between the film and a substrate is 80 MPa or more.

4. The biocompatible material according to any one of claims 1 to 3, wherein the biocompatible material has any one, two or three, or all of following characteristics:

   a) an arithmetic mean surface height Sa1 of surface roughness of the film is 2 $\mu$m or less;
   b) a difference between the arithmetic mean surface height Sa1 of the surface roughness of the film and an arithmetic mean surface height Sa2 of surface roughness of a surface which does not comprise the film is 300 nm or less;
   c) an arithmetic mean surface height Ra1 of line roughness of the film is 2 $\mu$m or less; and
   d) a difference between the arithmetic mean surface height Ra1 of the line roughness of the film and an arithmetic mean surface height Ra2 of line roughness of a surface which does not comprise the film is 300 nm or less.

5. The biocompatible material according to any one of claims 1 to 4, wherein an average thickness of the film is 0.10 to 30 $\mu$m.

6. The biocompatible material according to any one of claims 1 to 5, wherein the biocompatible material comprises a biocompatible substrate, and the biocompatible substrate is at least one selected from the group consisting of pure titanium, zirconia, a cobalt-chromium alloy, stainless steel, and a titanium alloy.

7. The biocompatible material according to any one of claims 1 to 6, wherein the biocompatible material is one selected from the group consisting of an artificial bone material, an intraosseous fixture material, a dental implant material, an orthodontic anchor screw material, a medullary nail material, and an interbody fixation material.

8. The biocompatible material according to any one of claims 1 to 7, wherein a shape of the biocompatible material is one selected from the group consisting of a cylindrical shape, a truncated cone shape, a conical shape, a shape having a screw-shaped threaded portion in a part of the shape, a rectangular parallelepiped and a cube, a block shape having a partially inclined surface, and a wedge shape.

9. A method of manufacturing a biocompatible material comprising a film consisting of magnesium and calcium, comprising the steps of:

(A) preparing a biocompatible substrate;
(B) preparing a sputtering target consisting of magnesium and calcium;
(C) cleaning a surface of the biocompatible substrate in vacuum; and
(D) sputtering by using the sputtering target and setting a temperature of the biocompatible substrate obtained in the Step (C) at 130°C or lower, to form the film consisting of magnesium and calcium on the biocompatible substrate, wherein a content of calcium in the film is more than 0 % by weight and 40 % by weight or less where a total weight of magnesium and calcium is 100 % by weight, and the film is free from $Mg_2Ca$.

10. The method according to claim 9, wherein
the film has any one, two, or all of following characteristics i) to iii):

i) hardness obtained by an indentation test is 0.4 GPa or more;
ii) a relationship Wc/t between a critical load Wc (N) and a film thickness t ($\mu$m) is 1 N/$\mu$m or more; and
iii) a critical shear stress at an interface between the film and a substrate is 80 MPa or more.

11. The method according to claim 9 or 10, wherein the method has any one, two, three, or all of following characteristics:

a) an arithmetic mean surface height Sa1 of surface roughness of the film is 2 $\mu$m or less;
b) a difference between the arithmetic mean surface height Sa1 of the surface roughness of the film and an arithmetic mean surface height Sa2 of surface roughness of a surface which does not comprise the film is 300 nm or less;
c) an arithmetic mean surface height Ra1 of line roughness of the film is 2 $\mu$m or less; and
d) a difference between the arithmetic mean surface height Ra1 of the line roughness of the film and an arithmetic mean surface height Ra2 of line roughness of a surface which does not comprise the film is 300 nm or less.

**Patentansprüche**

1. Biokompatibles Material mit einem Film bestehend aus Magnesium und Calcium,
wobei

der Calciumgehalt im Film mehr als 0 Gew.-% und
40 Gew.-% oder weniger beträgt, wobei das Gesamtgewicht an Magnesium und Calcium 100 Gew.-% beträgt, und der Film frei von $Mg_2Ca$ ist.

2. Biokompatible Material nach Anspruch 1, wobei der Film einen amorphen Anteil aufweist.

3. Biokompatible Material nach Anspruch 1 oder 2, wobei der Film eine, zwei oder alle der folgenden Eigenschaften i) bis iii) aufweist:

i) eine durch einen Eindringtest ermittelte Härte beträgt 0,4 GPa oder mehr;
ii) ein Verhältnis Wc/t zwischen einer kritischen Belastung Wc (N) und einer Filmdicke t ($\mu$m) beträgt 1 N/$\mu$m oder

mehr; und

iii) eine kritische Scherspannung an einer Grenzfläche zwischen dem Film und einem Substrat beträgt 80 MPa oder mehr.

4. Biokompatibles Material nach einem der Ansprüche 1 bis 3, wobei das biokompatible Material eine, zwei, drei oder alle der folgenden Eigenschaften aufweist:

a) eine arithmetische mittlere Oberflächenhöhe Sa1 der Oberflächenrauheit des Films beträgt 2 $\mu$m oder weniger;

b) eine Differenz zwischen der arithmetischen mittleren Oberflächenhöhe Sa1 der Oberflächenrauheit des Films und einer arithmetischen mittleren Oberflächenhöhe Sa2 der Oberflächenrauheit einer den Film nicht umfassenden Oberfläche beträgt 300 nm oder weniger;

c) eine arithmetische mittlere Oberflächenhöhe Ra1 der Linienrauheit des Films beträgt 2 $\mu$m oder weniger; und

d) eine Differenz zwischen der arithmetischen mittleren Oberflächenhöhe Ra1 der Linienrauheit des Films und einer arithmetischen mittleren Oberflächenhöhe Ra2 der Linienrauheit einer den Film nicht umfassenden Oberfläche beträgt 300 nm oder weniger.

5. Biokompatibles Material nach einem der Ansprüche 1 bis 4, wobei eine durchschnittliche Dicke des Films 0,10 bis 30 $\mu$m beträgt.

6. Biokompatibles Material nach einem der Ansprüche 1 bis 5, wobei das biokompatible Material ein biokompatibles Substrat umfasst und das biokompatible Substrat mindestens eines ist ausgewählt unter reinem Titan, Zirkondioxid, einer Kobalt-Chrom-Legierung, Edelstahl und einer Titanlegierung.

7. Biokompatibles Material nach einem der Ansprüche 1 bis 6, wobei das biokompatible Material ausgewählt ist unter einem künstlichen Knochenmaterial, einem intraossären Befestigungsmaterial, einem Zahnimplantatmaterial, einem Material für kieferorthopädische Verankerungsschrauben, einem Marknagelmaterial und einem Material zur Zwischenwirbelfixierung.

8. Biokompatibles Material nach einem der Ansprüche 1 bis 7, wobei die Form des biokompatiblen Materials ausgewählt ist unter einer zylindrischen Form, einer Kegelstumpfform, einer konischen Form, einer Form mit einem schraubenförmigen Gewindeabschnitt in einem Teil der Form, einem rechteckigen Parallelepiped und einem Würfel, einer Blockform mit einer teilweise geneigten Oberfläche und einer Keilform.

9. Verfahren zur Herstellung eines biokompatiblen Materials umfassend einen Film bestehend aus Magnesium und Calcium, mit den folgenden Schritten:

(A) Herstellen eines biokompatiblen Substrats;

(B) Herstellen eines aus Magnesium und Calcium bestehenden Sputtertargets;

(C) Reinigen einer Oberfläche des biokompatiblen Substrats im Vakuum; und

(D) Sputtern unter Verwendung des Sputtertargets und Einstellen einer Temperatur des in Schritt (C) erhaltenen biokompatiblen Substrats auf 130 °C oder weniger, um den aus Magnesium und Calcium bestehenden Film auf dem biokompatiblen Substrat zu bilden, wobei der Calciumgehalt im Film mehr als 0 Gew.-% und 40 Gew.-% oder weniger beträgt, wobei das Gesamtgewicht von Magnesium und Calcium 100 Gew.-% beträgt, und der Film frei von $Mg_2Ca$ ist.

10. Verfahren nach Anspruch 9, wobei der Film eine, zwei oder alle der folgenden Eigenschaften i) bis iii) aufweist:

i) eine durch einen Eindringtest ermittelte Härte beträgt 0,4 GPa oder mehr;

ii) ein Verhältnis Wc/t zwischen einer kritischen Belastung Wc (N) und einer Filmdicke t ($\mu$m) beträgt 1 N/$\mu$m oder mehr; und

iii) eine kritische Scherspannung an einer Grenzfläche zwischen dem Film und einem Substrat beträgt 80 MPa oder mehr.

11. Verfahren nach Anspruch 9 oder 10, wobei das Verfahren eine, zwei, drei oder alle der folgenden Eigenschaften aufweist:

a) eine arithmetische mittlere Oberflächenhöhe Sa1 der Oberflächenrauheit des Films beträgt 2 $\mu$m oder weniger;

b) eine Differenz zwischen der arithmetischen mittleren Oberflächenhöhe Sa1 der Oberflächenrauheit des Films und einer arithmetischen mittleren Oberflächenhöhe Sa2 der Oberflächenrauheit einer den Film nicht umfassenden Oberfläche beträgt 300 nm oder weniger;

c) eine arithmetische mittlere Oberflächenhöhe Ra1 der Linienrauheit des Films beträgt 2 $\mu$m oder weniger; und

d) eine Differenz zwischen der arithmetischen mittleren Oberflächenhöhe Ra1 der Linienrauheit des Films und einer arithmetischen mittleren Oberflächenhöhe Ra2 der Linienrauheit einer den Film nicht umfassenden Oberfläche beträgt 300 nm oder weniger.

**Revendications**

1. Matériau biocompatible présentant un film constitué de magnésium et de calcium,

dans lequel

la teneur en calcium du film est supérieure à 0 % en poids et inférieure ou égale à 40 % en poids, étant entendu que le poids total du magnésium et du calcium est de 100 % en poids, et le film est exempt de Mg$_2$Ca.

2. Matériau biocompatible selon la revendication 1, dans lequel le film présente une portion amorphe.

3. Matériau biocompatible selon la revendication 1 ou 2, dans lequel le film présente une, deux ou toutes les caractéristiques i) à iii) suivantes :

i) la dureté établie par un essai de pénétration est supérieure ou égale à 0,4 GPa,

ii) le rapport Wc/t entre la charge critique Wc (N) et l'épaisseur du film t ($\mu$m) est supérieur ou égal à 1 N/$\mu$m, et

iii) la contrainte de cisaillement critique à l'interface entre le film et un substrat est supérieure ou égale à 80 MPa.

4. Matériau biocompatible selon l'une quelconque des revendications 1 à 3, ledit matériau biocompatible présentant une, deux, trois ou toutes les caractéristiques suivantes :

a) la hauteur moyenne arithmétique Sa1 de rugosité de surface du film est inférieure ou égale à 2 $\mu$m,

b) la différence entre la hauteur moyenne arithmétique de surface Sa1 de rugosité de surface du film et la hauteur moyenne arithmétique de surface Sa2 de rugosité de surface d'une surface ne comprenant pas le film est inférieure ou égale à 300 nm,

c) la hauteur moyenne arithmétique de surface Ra1 de rugosité de profil du film est inférieure ou égale à 2 $\mu$m, et

d) la différence entre la hauteur moyenne arithmétique de surface Ra1 de rugosité de profil du film et la hauteur moyenne arithmétique de surface Ra2 de rugosité de profil d'une surface ne comprenant pas le film est inférieure ou égale à 300 nm.

5. Matériau biocompatible selon l'une quelconque des revendications 1 à 4, dans lequel l'épaisseur moyenne du film est de 0,10 à 30 $\mu$m.

6. Matériau biocompatible selon l'une quelconque des revendications 1 à 5, ledit matériau biocompatible comprenant un substrat biocompatible, et le substrat biocompatible étant au moins un matériau choisi dans le groupe constitué par le titane pur, le zircone, un alliage de cobalt-chrome, l'acier inoxydable et un alliage de titane.

7. Matériau biocompatible selon l'une quelconque des revendications 1 à 6, ledit matériau biocompatible étant un matériau choisi dans le groupe constitué par un matériau osseux artificiel, un matériau de fixation intra-osseuse, un matériau d'implant dentaire, un matériau de vis d'ancrage orthodontique, un matériau de clou médullaire et un matériau de fixation intersomatique.

8. Matériau biocompatible selon l'une quelconque des revendications 1 à 7, dont la forme est choisie dans le groupe constitué par une forme cylindrique, une forme de cône tronqué, une forme conique, une forme ayant une portion filetée en forme de vis dans une partie de la forme, un parallélépipède rectangle et un cube, une forme de pavé ayant une surface partiellement inclinée, et une forme de coin.

9. Procédé de fabrication d'un matériau biocompatible comprenant un film constitué de magnésium et de calcium, comprenant les étapes consistant à :

(A) préparer un substrat biocompatible,

(B) préparer une cible de pulvérisation constituée de magnésium et de calcium,

(C) nettoyer une surface du substrat biocompatible sous vide, et

(D) effectuer une pulvérisation au moyen de la cible de pulvérisation et avec un réglage de la température du substrat biocompatible obtenu à l'étape (C) à 130 °C ou moins, afin de former le film constitué de magnésium et de calcium sur le substrat biocompatible, la teneur en calcium du film étant supérieure à 0 % en poids et inférieure ou égale à 40 % en poids, étant entendu que le poids total du magnésium et du calcium est de 100 % en poids, et le film étant exempt de $Mg_2Ca$.

**10.** Procédé selon la revendication 9, dans lequel le film comprend une, deux ou toutes les caractéristiques i) à iii) suivantes :

i) la dureté établie par un essai de pénétration est supérieure ou égale à 0,4 GPa,

ii) le rapport Wc/t entre la charge critique Wc (N) et l'épaisseur du film t ($\mu$m) est supérieur ou égal à 1 N/$\mu$m, et

iii) la contrainte de cisaillement critique à l'interface entre le film et un substrat est supérieure ou égale à 80 MPa.

**11.** Procédé selon la revendication 9 ou 10, ledit procédé comprenant une, deux, trois ou toutes les caractéristiques suivantes :

a) la hauteur moyenne arithmétique de surface Sa1 de rugosité de surface du film est inférieure ou égale à 2 $\mu$m,

b) la différence entre la hauteur moyenne arithmétique de surface Sa1 de rugosité de surface du film et la hauteur moyenne arithmétique de surface Sa2 de rugosité de surface d'une surface ne comprenant pas le film est inférieure ou égale à 300 nm,

c) une hauteur moyenne arithmétique de surface Ra1 de rugosité de profil du film est inférieure ou égale à 2 $\mu$m, et

d) la différence entre la hauteur moyenne arithmétique de surface Ra1 de rugosité de profil du film et la hauteur moyenne arithmétique de surface Ra2 de rugosité de profil d'une surface ne comprenant pas le film est inférieure ou égale à 300 nm.

Fig. 1

Fig. 2

Fig. 3

Fig. 4

Fig. 5

Fig. 6

AC1-C0        AC1-C10

AC1-C20        AC1-C30

Fig. 7

A1-C0        A1-C10

A1-C20        A1-C30

Fig. 8

A2-C0

A2-C10

A2-C20

A2-C30

Fig. 9

Fig. 10

Fig. 11

Fig. 12

Fig. 13

A1-C0    A1-C10

A1-C20    A1-C30

Fig. 14

A2-C0    A2-C10

A2-C20    A2-C30

Fig. 15

Fig. 16

Fig. 17

Fig. 18

Fig. 19

A1-C0

A1-C10

A1-C20

A1-C30

Fig. 20

A2-C0

A2-C10

A2-C20

A2-C30

Fig. 21

Fig. 22

Fig. 23

Fig. 24

Fig. 25

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009147819 A
- JP 4425198 B
- JP HEI32540 B
- WO 2016189099 A1
- WO 2020099334 A2
- EP 1847278 A1
- WO 2008035948 A1

**Non-patent literature cited in the description**

- *POIEX/HACEX Catalogue*
- **KYOUSUKE UEDA**. *Materia Japan*, 2012, vol. 51 (9)
- *Implant Journal*, 2017, 8
- **X. LI et al.** *Scientific Reports*, 2017, vol. 7, 40755
- **J. HOFSTETER et al.** *JOM*, 2014, vol. 68 (4), 566-572
- **S. PAUL**. *Materialia*, 2020, S25
- **K. SAKSL et al.** *J. Alloys and Compounds*, 2019, vol. 801, 651-657
- **J. LIU et al.** *J. Alloys and Compounds*, 2018, vol. 742, 524-535
- **J. LI et al.** *Chemical Communications*, 2017, vol. 53, 8288-8291
- **G.E. DIETER**. Mechanical metallurgy. McGraw-Hill, 1988
- **K. L. JOHNSON**. Contact mechanics. Cambridge university press, 1985